# EUROPÄISCHE PATENTSCHRIFT

(11) **EP 3 606 505 B1**
(45) Veröffentlichungstag und Bekanntmachung des Hinweises auf die Patenterteilung: **06.01.2021**
(21) Anmeldenummer: 18721711.2
(22) Anmeldetag: 20.04.2018
(51) Int. Cl.: A61K 9/00, A61P 11/02, A61K 47/36, A61K 45/06, A61K 31/137, A61K 31/4174, A61K 31/728

(54) **ZUSAMMENSETZUNG FÜR DIE NASALE APPLIKATION**
COMPOSITION FOR NASAL APPLICATION
COMPOSITION POUR APPLICATION NASALE

(30) Priorität: 31.07.2017 DE 102017007144; 01.08.2017 DE 102017007171; 08.08.2017 DE 102017117987
(43) Veröffentlichungstag der Anmeldung: 12.02.2020
(73) Patentinhaber: Maria Clementine Martin Klosterfrau Vertriebsgesellschaft mbH, 50670 Köln (DE)
(72) Erfinder: UNKAUF, Markus, 80538 München (DE); MIETHING, Holger, 12107 Berlin (DE)
(74) Vertreter: Strehlke, Ingo Kurt
(86) Internationale Anmeldenummer: PCT/EP2018/060132
(87) Internationale Veröffentlichungsnummer: WO 2019/025040

(56) Entgegenhaltungen:
- WO-A1-03/049747
- DE-U1- 20 318 634
- DE-U1-202012 002 792
- DE-U1-202012 005 650
- Claudia Sikora: "Einfluss von Rezepturverbesserungen auf die Zytotoxizit?t von Nasalia", , 14. Juli 2006 (2006-07-14), XP055108889, Gefunden im Internet: URL:http://ub-ed.ub.uni-greifswald.de/opus /volltexte/2006/128/pdf/sikora_claudia.pdf [gefunden am 2014-03-19]
- MÖSGES RALPH ET AL: "Dexpanthenol: An Overview of its Contribution to Symptom Relief in Acute Rhinitis Treated with Decongestant Nasal Sprays", ADVANCES IN THERAPY, HEALTH COMMUNICATIONS, METUCHEN, NJ, US, Bd. 34, Nr. 8, 10. Juli 2017 (2017-07-10), Seiten 1850-1858, XP036302767, ISSN: 0741-238X, DOI: 10.1007/S12325-017-0581-0 [gefunden am 2017-07-10]

## Beschreibung

Die vorliegende Erfindung betrifft das medizinisch-therapeutische Gebiet der Behandlung von Rhinitiden.

Insbesondere betrifft die vorliegende Erfindung eine Zusammensetzung, insbesondere eine pharmazeutische Zusammensetzung, welche sich vorzugsweise für die topische, insbesondere nasale, bevorzugt intranasale Applikation eignet, insbesondere für die Behandlung von Rhinitiden.

Zudem betrifft die vorliegende Erfindung auch eine diese Zusammensetzung enthaltende Applikationsvorrichtung sowie eine entsprechende Verpackungseinheit.

Weiterhin sind vorliegend auch Verwendungen der Zusammensetzung nach der vorliegenden Erfindung im Rahmen der prophylaktischen bzw. kurativen topischen Behandlung von Rhinitis beschrieben.

Die vorliegende Erfindung betrifft auch Hyaluronsäure bzw. physiologisch verträgliches Hyaluronsäure-Salz (Hyaluronat) zur Verwendung bei der topischen Behandlung von Rhinitis sowie Verfahren zur Stabilisierung bzw. Erhöhung der Stabilität bzw. zur Verbesserung der Verträglichkeit bzw. zur Erhöhung der Wirksamkeit einer diesbezüglichen Zusammensetzung, insbesondere pharmazeutischen Zusammensetzung.

In diesem Zusammenhang betrifft die vorliegende Erfindung auch ein Verfahren zur Stabilisierung bzw. zur Verbesserung der Verträglichkeit bzw. zur Erhöhung der Wirksamkeit eines Wirkstoffs (a) bzw. einer Komponente (a) und/oder eines Wirkstoffs (b) bzw. einer Komponente (b) in einer entsprechenden Zusammensetzung, insbesondere pharmazeutischen Zusammensetzung.

Schließlich betrifft die vorliegende Erfindung auch Verwendungen von Hyaluronsäure bzw. eines physiologisch verträglichen Hyaluronsäure-Salzes zur Stabilisierung bzw. zur Verbesserung der Verträglichkeit bzw. zur Erhöhung der Wirksamkeit einer diesbezüglichen Zusammensetzung, insbesondere pharmazeutischen Zusammensetzung.

Unter einer Rhinitis (synonym gelegentlich auch als Nasenkatarrh, Schnupfen oder Koryza bezeichnet) versteht man im Rahmen der vorliegenden Erfindung insbesondere eine akute oder chronische Entzündung der Nasenschleimhäute, wobei die Rhinitis insbesondere infektiösen (z. B. viralen oder bakteriellen), allergischen oder pseudoallergischen Ursprungs sein kann. Am Häufigsten tritt eine Rhinitis im Rahmen einer sogenannten Erkältung auf.

Neben einer Unterscheidung von akuter Rhinitis einerseits und chronischer Rhinitis andererseits unterscheidet man auch verschiedene Formen von Rhinitiden, so beispielsweise die folgenden Rhinitisformen: *Rhinitis acuta, Rhinitis atrophicans, Rhinitis allergica, Rhinitis hypertrophica, Rhinitis medicamentosa, Rhinitis pseudomembranacea, Rhinitis sicca, Rhinitis vasomotorica* und die umweltbedingte Rhinitis.

Bei der sogenannten akuten Rhinitis (*Rhinitis acuta*), d. h. dem gewöhnlichen Schnupfen, handelt es sich in der Regel um einen im Allgemeinen harmlosen Infekt der Nasenschleimhäute und damit um eine infektiöse Rhinitis, welche meist viral, insbesondere durch eine Vielzahl von Viren (insbesondere Rhinoviren und/oder Adenoviren), gelegentlich aber auch bakteriell ausgelöst werden kann. Hauptmerkmal einer akuten Rhinitis ist eine sogenannte laufende Nase und eine Verstopfung der Nase durch die Anschwellung der Schleimhäute.

Insgesamt sind mehr als 200 "Schnupfenviren" als mögliche Auslöser einer viralen Rhinitis bekannt, wie sie üblicherweise im Rahmen einer gewöhnlichen Erkältung auftreten kann. Im Rahmen einer Erkältung, welche im Allgemeinen mit einer Rhinitis beginnt, verschwindet aber die *Rhinitis acuta* im Allgemeinen. Gelegentlich kann es jedoch mitunter auch zu einer Chronifizierung kommen, welche oftmals mit einer Volumenzunahme der Schleimhäute unter anderem im Bereich der Nasenmuscheln mit Behinderung der Nasenatmung einhergeht.

Eine Rhinitis (d. h. eine Entzündung der Nasenschleimhaut), insbesondere eine akute Rhinitis, kann oftmals gleichzeitig auch mit einer Entzündung der Schleimhaut der Nasennebenhöhlen (Sinusitis) vorliegen; in einem solchen Fall spricht man auch von einer Rhinosinusitis. Auch kann eine Rhinitis, insbesondere eine akute Rhinitis, gleichzeitig auch mit einer Entzündung des Rachens (Pharyngitis) vorliegen; in einem solchen Fall spricht man auch von einer Rhinopharyngitis.

Für weitergehende Einzelheiten zum Begriff der Rhinitis kann insbesondere verwiesen werden auf Pschyrembel, Medizinisches Wörterbuch, 257. Auflage, Seiten 1331/1332, insbesondere Stichwörter "*Rhinitis*", "*Rhinitis allergica*", "*Rhinitis atrophicans", "Rhinitis hyperplastica*", "*Rhinitis pseudomembranacea", "Rhinitis sicca*" und "*Rhinitis vasomotorica*".

Da es also eine Vielzahl verschiedener Typen von Viren gibt, welche eine akute virale Rhinitis auslösen können, und da eine Vielzahl von Ursachen für das Auftreten einer Rhinitis existieren, können Rhinitiden, insbesondere akute Rhinitiden, im Allgemeinen nicht kausal, sondern nur symptomatisch, insbesondere topisch, behandelt werden.

Zu diesem Zweck kommen in den meisten Fällen intranasal zu applizierende Zusammensetzungen zur Anwendung, welche sogenannte Sympathomimetika (synonym auch als "Abschweller", "Dekongestiva" oder dergleichen bezeichnet), vorzugweise alpha-Sympathomimetika (wie z. B. Xylometazolin und Oxymetazolin oder deren physiologisch verträgliche bzw. unbedenkliche Salze), enthalten.

Die vorgenannten Sympathomimetika führen aber insbesondere im Fall längerer Anwendungsdauern zu Verträglichkeitsproblemen. So ist es bekannt, dass diese Sympathomimetika zwar aufgrund ihrer vasokonstriktorischen Eigenschaften nach lokaler bzw. topischer Anwendung in der Nase zunächst zu einer Nasenschleimhautabschwellung führen, jedoch bei wiederholten Anwendung oftmals eine Austrocknung der Nasenschleimhäute bewirken, einhergehend mit entzündlichen Irritationen der Nasenschleimhäute (was nicht selten zu einer erhöhten Infektionsgefahr führt, da die Nasenschleimhaut im ausgetrockneten und entzündeten Zustand nicht mehr ihre Schutz- und Filterfunktion im vollen Umfang aufrechterhalten kann und folglich Krankheitserreger ungehindert in die Atemwege gelangen können). Darüber hinaus haben die Sympathomimetika zum Teil auch schwerwiegende systemische (z. B. kardiovaskuläre) Nebenwirkungen, insbesondere im Fall von Überdosierung oder bei der Therapierung bestimmter Patienten (z. B. Kleinkindern, Patienten mit kardiovaskulären Vorerkrankungen etc.). Weiterhin besteht bei dem Einsatz von Sympathomimetika das Problem, dass diese bei häufiger oder längerfristiger Anwendung zu einer gewissen Abhängigkeit führen, da die Nasenschleimhaut nur noch unter Verwendung von Sympathomimetika und nicht mehr von alleine abschwillt. Dies äußert sich dann als dauerhaft "verstopfte" Nase, was auch als *Rhinitis medicamentosa* bezeichnet wird.

Um den zahlreichen Nebenwirkungen der Sympathomimetika zumindest teilweise entgegenzuwirken, werden in für die intranasale Applikation bestimmte sympathomimetikahaltige Zusammensetzungen bisweilen weitere Wirk- bzw. Inhaltsstoffe inkorporiert, beispielsweise pflanzliche Extrakte oder pflanzliche Inhaltsstoffe, befeuchtende oder schleimhautpflegende Zusätze, Antihistaminika, Salze etc. (vgl. beispielsweise DE 195 41 919 A1, DE 195 49 421 A1 und DE 103 56 248 A1).

Die DE 20 2012 002 792 U1 betrifft eine Zusammensetzung für die Behandlung von Rhinitiden, wobei die Zusammensetzung mindestens ein imidazolinbasiertes alpha-Sympathomimetikum oder dessen physiologisch unbedenkliches Salz sowie Pantothenol oder dessen physiologisch unbedenkliche Ester bzw. Pantothensäure oder deren physiologisch unbedenkliche Salze enthält, wobei die Zusammensetzung weiterhin einen speziellen pH-Wert aufweisen soll. Zudem wird auf den fakultativen Einsatz von Hyaluronsäure bzw. eines Hyaluronsäure-Salzes abgestellt, wobei jedoch keine Spezifizierung des Molekulargewichts erfolgt.

Weiterhin betrifft die DE 20 2012 005 650 U1 die Verwendung von Pantothenol oder dessen physiologisch unbedenklichen Estern bzw. Pantothensäure oder deren physiologisch unbedenklichen Salzen zusammen bzw. in Kombination mit mindestens einem imidazolinbasierten alpha-Sympathomimetikum oder dessen physiologisch unbedenklichen Salzen zur Verringerung der systemischen Resorption des imidazolinbasierten alpha-Sympathomimetikums bei der topischen Behandlung von Rhinitiden bzw. zur Herstellung eines Arzneimittels zur Behandlung von Rhinitiden mittels nasaler Applikation sowie ein diesbezügliches Kombinationstherapeutikum als solches.

Darüber hinaus betrifft die DE 203 18 634 U1 eine pharmazeutische Zusammensetzung zur Behandlung von Rhinitiden, welche mindestens ein zur topischen Anwendung geeignetes Sympathomimetikum sowie mindestens ein vorzugsweise saures Glykosaminoglykan oder dessen physiologisch unbedenkliche Salze, insbesondere Hyaluronsäure oder deren Salze, enthält.

Weiterhin betrifft das Dokument gemäß der Dissertationsschrift "*Einfluss von Rezepturverbesserung auf die Cytotoxicität von Nasalia, Claudia Sikora"* allgemeine Ausführungen bzw. Untersuchungen zu einer Vielzahl von anwendbaren Arzneimitteln und Medizinprodukten bzw. von Nasalia, wobei in diesem Zusammenhang maßgeblich auf den Einfluss von Konservierungsmitteln insbesondere im Hinblick auf deren Verträglichkeit bei der Anwendung der dort untersuchten Zusammensetzungen abgestellt wird.

Zudem betrifft das Dokument gemäß der wissenschaftlichen Publikation Mösges et al.: "Dexpanthenol: An Overview of its Contribution to Symptom Relief in Acute Rhinitis Treated with Decongestant Nasal Sprays", Adv Ther. 2017, Seiten 1850 bis 1858 einen Übersichtsartikel zu Dexpanthenol und seiner Verwendung zur Behandlung akuter Rhinitis bzw. in abschwellenden Nasensprays.

Darüber hinaus sind aus dem Stand der Technik für die Behandlung von Rhinitiden auch sympathomimetikafreie, für die intranasale Applikation bestimmte Zusammensetzungen bekannt, z. B. Zusammensetzungen auf Basis ätherischer Öle, welche aber oftmals keine ausreichende Wirksamkeit in Bezug auf eine effiziente Behandlung von Rhinitiden aufweisen. Darüber hinaus weisen derartige Zusammensetzungen ebenfalls nicht immer eine optimale Verträglichkeit auf. Insbesondere pflanzliche Stoffe, wie beispielsweise ätherische Öle, können zu Schleimhautreizungen bzw. Unverträglichkeitsreaktionen führen.

Die WO 03/049747 A1 betrifft eine pharmazeutische Zusammensetzung, die wenigstens Panthenol und/oder Pantothensäure und Hyaluronsäure und/oder Hyaluronat sowie gegebenenfalls zusätzlich pharmazeutische Hilfsmittel umfassen soll, wobei die Hyaluronsäure bzw. das Hyaluronat ein Molekulargewicht in einem Bereich von etwa 50.000 bis etwa 10.000.000 Dalton aufweisen soll.

Zudem zeichnen sich zahlreiche, aus dem Stand der Technik bekannte Zusammensetzungen, welche im Rahmen der Behandlung von Rhinitiden verabreicht werden, lediglich durch kurze Wirkdauern aus.

Darüber hinaus sind diese Sympathomimetika, insbesondere Xylometazolin und Oxymetazolin sowie deren physiologisch verträglichen Salze, nicht unbegrenzt in wässriger Lösung lagerbar, so dass unerwünscht und unkontrolliert Abbauprodukte dieser Wirkstoffe entstehen können, welche dann einer Anwendung entsprechender wässriger Lösungen dieser Wirkstoffe entgegenstehen können.

So ist es beispielsweise für das alpha-Sympathomimetikum Xylometazolinhydrochlorid bekannt, dass es in wässriger Lösung hydrolytisch gespalten werden kann. Wie die nachfolgende Reaktionsgleichung zeigt, wird Xylometazolinhydrochlorid (1) in wässriger Lösung hydrolytisch unter Öffnung des Imidazolinrings gespalten, so dass als Abbauprodukt das Amid (2) gebildet wird, welches in der Monographie des Europäischen Arzneibuchs (Ph. Eur.) auch als sogenannte "Verunreinigung A" bezeichnet wird:

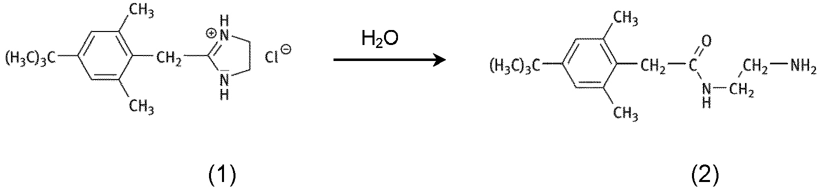

Für das Oxymetazolinhydrochlorid gilt ein analoger Mechanismus, welcher durch die folgende Reaktionsgleichung wiedergegeben werden kann, wobei das Oxymetazolinhydrochlorid (3) hydrolytisch zu dem Amid (4) als Abbauprodukt umgewandelt wird:

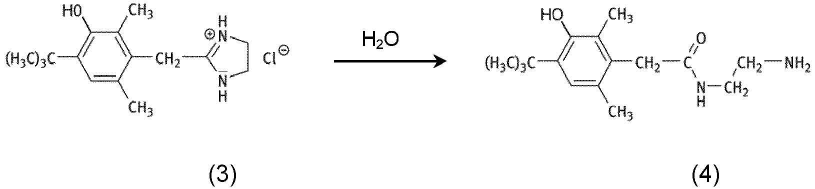

Um einem unerwünschten Abbau der Abschweller auf Sympathomimetikumbasis, insbesondere auf Basis von Xylometazolin und/oder Oxymetazolin sowie deren Salzen, entgegenzuwirken, wurden im Stand der Technik verschiedene Maßnahmen ergriffen, welche sich aber als unzulänglich erwiesen haben bzw. mit unerwünschten Nachteilen verbunden sind:
So führt die Zugabe externer Stabilisatoren oftmals zu einer nur unzureichenden Stabilisierung dieser Wirkstoffe. Eine Zugabe komplexierender Salze, insbesondere von Zinksalzen, führt zwar zu einer gewissen Stabilitätsverbesserung (vgl. DE 103 37 186 A1 und WO 2005/018601 A1), führt jedoch bei nasaler Applikation zu einer unerwünschten Reizung der Nasenschleimhäute infolge der Zinksalze, einhergehend mit entzündlichen Irritationen.

Daher können die im Stand der Technik aufgezeigten Versuche bzw. Lösungsansätze zur Stabilisierung der vorgenannten sympathomimetikabasierten Wirkstoffe in wässrigen Systemen nur als praktizierter Kompromiss in Ermangelung einer besseren Alternative angesehen werden. Zudem berücksichtigen zu vorgenannten Lösungsansätze nicht den austrocknenden Effekt dieser Wirkstoffe in Bezug auf die Nasenschleimhäute.

Um andererseits einer Austrocknung der Nasenschleimhäute durch die Anwendung von alpha-Sympathomimetika entgegenzuwirken, wurde im Stand der Technik die kombinierte Verabreichung von Pantothenol und/oder Pantothensäure (vgl. die zu derselben Patentfamilie gehörenden Druckschriften DE 195 41 919 A1, DE 195 49 421 A1 und EP 1 663 141 B1), da diese Wirkstoffe (d. h. Pantothenol bzw. Pantothensäure und deren jeweils physiologisch verträgliche Salze) einer Austrocknung der Nasenschleimhäute durch die Anwendung der alpha-Sympathomimetika in effizienter Weise entgegenwirken. In diesem Zusammenhang hat sich insbesondere die Anwendung von Pantothenol bewährt.

Insbesondere der Wirkstoff Pantothenol (synonym auch als "Dexpanthenol" bezeichnet) kann jedoch in wässriger Lösung hydrolytisch gemäß der nachfolgenden Reaktionsgleichung gespalten werden, wobei sich aus dem Wirkstoff Panthenol (3) in saurer Lösung - neben dem Ammoniumsalz des 3-Aminopropanols (7) - das Lacton D-Pantolacton (6) als Abbauprodukt bildet, während in alkalischer Lösung - neben 3-Aminopropanol (5) - das Salz der Pantosäure (4) gebildet wird:

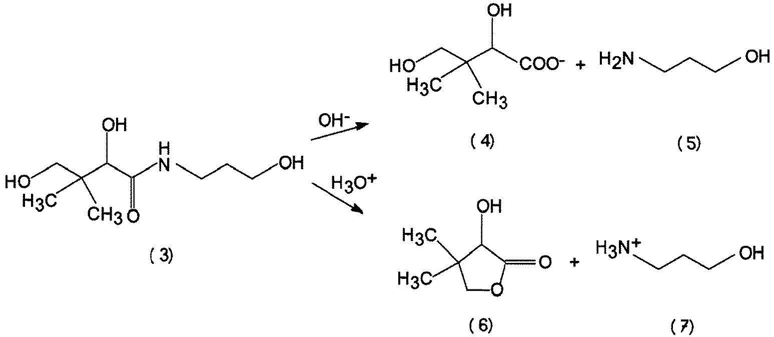

In gemeinsamer wässriger Lösung von alpha-Sympathomimetika, insbesondere auf Basis von Xylometazolin und/oder Oxymetazolin, einerseits und Panthenol bzw. Pantothensäure andererseits ist eine Stabilisierung der einzelnen Inhaltsstoffe besonders diffizil, da die einzelnen Wirkstoffe (d. h. Xylometazolin bzw. Oxymetazolin einerseits und Panthenol bzw. Dexpanthenol andererseits) in unterschiedlichen pH-Bereichen ihr Stabilitätsoptimum aufweisen.

Die der vorliegenden Erfindung zugrundeliegende Aufgabe besteht daher in der Bereitstellung einer vorzugsweise für die topische, insbesondere nasale, bevorzugt intranasale Applikation, insbesondere für die Behandlung von Rhinitis (Rhinitiden), geeigneten Zusammensetzung, insbesondere pharmazeutischen Zusammensetzung, welche die zuvor geschilderten Nachteile des Standes der Technik zumindest weitgehend vermeidet oder aber wenigstens abschwächt.

Insbesondere soll eine vorzugsweise für die topische, insbesondere nasale, bevorzugt intranasale Applikation, insbesondere für die Behandlung von Rhinitis (Rhinitiden), geeignete Zusammensetzung, insbesondere pharmazeutischen Zusammensetzung, bereitgestellt werden, welche eine verbesserte Stabilität (z. B. Lager- bzw. Lagerungsstabilität) aufweist. Insbesondere soll eine solche Zusammensetzung auch nach längerer Lagerung keine bzw. keine signifikanten Mengen an Abbauprodukten der Inhaltsstoffe, insbesondere keine signifikanten Mengen an Abbauprodukten des Sympathomimetikums, nämlich des Xylometazolins, und/oder des Panthenols aufweisen.

Des Weiteren soll eine vorzugsweise für die topische, insbesondere nasale, bevorzugt intranasale Applikation, insbesondere für die Behandlung von Rhinitiden, geeignete Zusammensetzung, insbesondere pharmazeutischen Zusammensetzung, bereitgestellt werden, welche gegenüber herkömmlichen, für die Behandlung von Rhinitiden mittels topischer bzw. intranasaler Applikation bestimmten pharmazeutischen Präparaten eine verbesserte Wirkeffizienz und/oder eine erhöhte bzw. verlängerte Wirksamkeit bzw. Wirkdauer und/oder eine verbesserte Verträglichkeit, insbesondere ein verbessertes Nebenwirkungsprofil, aufweist. Darüber hinaus soll insbesondere auch die Wirkdauer verlängert sein.

Zur Lösung der zuvor geschilderten Aufgabe schlägt die vorliegende Erfindung - gemäß einem **ersten** Aspekt der vorliegenden Erfindung - eine Zusammensetzung nach Anspruch 1 vor; weitere, insbesondere vorteilhafte Ausgestaltungen der erfindungsgemäßen Zusammensetzung sind Gegenstand der diesbezüglichen, die Zusammensetzung betreffenden Unteransprüche.

Weiterhin betrifft die vorliegende Erfindung - gemäß einem **zweiten** Aspekt der vorliegenden Erfindung - eine die erfindungsgemäße Zusammensetzung enthaltende Applikationsvorrichtung gemäß dem diesbezüglich unabhängigen Vorrichtungsanspruch.

Die vorliegende Erfindung betrifft gleichermaßen - gemäß einem **dritten** Aspekt der vorliegenden Erfindung - auch die erfindungsgemäße Verpackungseinheit, wie sie in dem diesbezüglich unabhängigen Patentanspruch definiert ist.

Zudem betrifft die vorliegende Erfindung - gemäß einem **vierten** Aspekt der vorliegenden Erfindung - auch Hyaluronsäure bzw. physiologisch verträgliches Hyaluronsäure-Salz (Hyaluronat) zur Verwendung bei der topischen Behandlung von Rhinitis gemäß dem diesbezüglich unabhängigen Patentanspruch.

Weiterhin betrifft die vorliegende Erfindung - gemäß einem **fünften** Aspekt der vorliegenden Erfindung - das erfindungsgemäße Verfahren zur Stabilisierung bzw. zur Verbesserung der Verträglichkeit bzw. zur Erhöhung der Wirksamkeit einer Zusammensetzung, insbesondere pharmazeutischen Zusammensetzung, wie es in dem diesbezüglich unabhängigen Verfahrensanspruch definiert ist. Gemäß diesem Aspekt der vorliegenden Erfindung betrifft die vorliegende Erfindung gleichermaßen auch ein Verfahren zur Stabilisierung bzw. zur Verbesserung der Verträglichkeit bzw. zur Erhöhung der Wirksamkeit einer Komponente (a) bzw. einer Komponente (b) gemäß dem diesbezüglich unabhängigen Verfahrensanspruch.

Weiterhin betrifft die vorliegende Erfindung - gemäß einem **sechsten** Aspekt der vorliegenden Erfindung - die erfindungsgemäßen Verwendungen von Hyaluronsäure bzw. eines physiologisch verträglichen Hyaluronsäure-Salzes (Hyaluronat) zur Stabilisierung bzw. zur Verbesserung der Verträglichkeit bzw. zur Erhöhung der Wirksamkeit einer Zusammensetzung, insbesondere pharmazeutischen Zusammensetzung, gemäß den diesbezüglichen unabhängigen Verwendungsansprüchen; weitere, insbesondere vorteilhafte Ausgestaltungen sind Gegenstand des diesbezüglichen Unteranspruchs.

Es versteht sich im Zusammenhang mit den nachfolgenden Ausführungen von selbst, dass Ausgestaltungen, Ausführungsformen, Vorteile und dergleichen, welche nachfolgend zu Zwecken der Vermeidung von Wiederholungen nur zu einem Erfindungsaspekt aufgeführt sind, selbstverständlich auch in Bezug auf die übrigen Erfindungsaspekte entsprechend gelten, ohne dass dies einer gesonderten Erwähnung bedarf.

Bei allen nachstehend genannten relativen bzw. prozentualen gewichtsbezogenen Angaben, insbesondere Mengenangaben, ist weiterhin zu beachten, dass diese im Rahmen der vorliegenden Erfindung vom Fachmann derart auszuwählen sind, dass sie sich in der Summe unter Einbeziehung aller Komponenten bzw. Inhaltsstoffe, insbesondere wie nachfolgend definiert, stets zu 100% bzw. 100 Gew.-% ergänzen bzw. addieren; dies versteht sich aber für den Fachmann von selbst.

Weiterhin gilt, dass der Fachmann - anwendungsbezogen oder einzelfallbedingt - von den nachfolgend angeführten Gewichts-, Mengen- und Bereichsangaben abweichen kann, ohne dass er den Rahmen der vorliegenden Erfindung verlässt.

Dies vorausgeschickt, wird die vorliegende Erfindung nunmehr nachfolgend im Detail erläutert.

Gegenstand der vorliegenden Erfindung - gemäß einem **ersten** Aspekt der vorliegenden Erfindung - ist eine Zusammensetzung, insbesondere pharmazeutische Zusammensetzung, vorzugsweise für die topische, insbesondere nasale, bevorzugt intranasale Applikation, insbesondere für die Behandlung von Rhinitis, wobei die Zusammensetzung in Kombination und jeweils in wirksamen, insbesondere pharmazeutisch wirksamen Mengen enthält:
(a) Xylometazolin, insbesondere in Form von dessen physiologisch verträglichen Salzen, besonders bevorzugt Xylometazolinhydrochlorid (Komponente (a));
(b) Pantothenol oder dessen physiologisch unbedenkliche Ester (Komponente (b));
(c) Hyaluronsäure und/oder ein physiologisch verträgliches Hyaluronsäure-Salz (Hyaluronat), insbesondere in Form eines Alkalisalzes der Hyaluronsäure, besonders bevorzugt in Form eines Natriumsalzes der Hyaluronsäure (Komponente (c)),
   wobei die Hyaluronsäure und/oder das physiologisch verträgliche Hyaluronsäure-Salz (Komponente (c)) ein zahlenmittleres Molekulargewicht (Molmasse) Mₙ im Bereich von 0,5 MDa bis 5 MDa und/oder ein gewichtsmittleres Molekulargewicht (Molmasse) M_{w} im Bereich von 0,6 MDa bis 6 MDa aufweist.

In diesem Zusammenhang betrifft die vorliegende Erfindung gemäß diesem Aspekt auch eine Zusammensetzung, insbesondere pharmazeutische Zusammensetzung, vorzugsweise für die topische, insbesondere nasale, bevorzugt intranasale Applikation, insbesondere für die Behandlung von Rhinitis, insbesondere *Rhinitis acuta*, insbesondere wie zuvor definiert,
wobei die Zusammensetzung in Kombination und jeweils in wirksamen, insbesondere pharmazeutisch wirksamen Mengen enthält:
(a) Xylometazolin, insbesondere in Form von dessen physiologisch verträglichen Salzen, besonders bevorzugt Xylometazolinhydrochlorid (Komponente (a));
(b) Pantothenol oder dessen physiologisch unbedenkliche Ester, insbesondere Pantothenol, besonders bevorzugt Dexpanthenol (Komponente (b));
(c) Hyaluronsäure und/oder ein physiologisch verträgliches Hyaluronsäure-Salz (Hyaluronat), insbesondere in Form eines Alkalisalzes der Hyaluronsäure, besonders bevorzugt in Form eines Natriumsalzes der Hyaluronsäure (Komponente (c)),
   wobei die Hyaluronsäure und/oder das physiologisch verträgliche Hyaluronsäure-Salz (Komponente (c)) ein zahlenmittleres Molekulargewicht (Molmasse) Mₙ im Bereich von 0,5 MDa bis 5 MDa und/oder ein gewichtsmittleres Molekulargewicht (Molmasse) M_{w} im Bereich von 0,6 MDa bis 6 MDa aufweist.

Wie die Anmelderin vollkommen überraschend herausgefunden hat, sind mit der erfindungsgemäßen Zusammensetzung eine Vielzahl von Vorteilen und Besonderheiten verbunden, wobei die nachfolgenden diesbezüglichen Ausführungen keinerlei Anspruch auf Vollständigkeit erheben.

So weist die erfindungsgemäße Zusammensetzung insbesondere eine verbesserte Stabilität (z. B. Lager- bzw. Lagerungsstabilität) auf. Insbesondere weist die Zusammensetzung nach der vorliegenden Erfindung auch nach längerer Lagerung keine bzw. keine signifikanten Mengen an Abbauprodukten der Inhaltsstoffe, insbesondere keine signifikanten Mengen an Abbauprodukten des Sympathomimetikums, nämlich des Xylometazolins, und/oder des Panthenols (Pantothenols) auf.

Darüber hinaus weist die Zusammensetzung nach der vorliegenden Erfindung überraschenderweise eine gegenüber herkömmlichen, für die Behandlung von Rhinitiden mittels topischer bzw. intranasaler Applikation bestimmten pharmazeutischen Präparaten eine verbesserte Wirkeffizienz und eine erhöhte bzw. verlängerte Wirksamkeit bzw. Wirkdauer und darüber hinaus auch eine verbesserte Verträglichkeit, insbesondere ein verbessertes Nebenwirkungsprofil, auf. Insbesondere ist auch die Wirkdauer der Zusammensetzung nach der vorliegenden Erfindung gegenüber herkömmlichen, für die Behandlung von Rhinitiden mittels topischer bzw. intranasaler Applikation bestimmten pharmazeutischen Präparaten verlängert.

In diesem Zusammenhang kommt der erfindungsgemäß eingesetzten Hyaluronsäure bzw. dem erfindungsgemäß eingesetzten physiologisch verträglichen Hyaluronsäure-Salz mit dem diesbezüglich sehr speziellen Molekulargewicht (synonym auch als Molmasse bezeichnet), wonach nämlich erfindungsgemäß relativ hohe Molekulargewichte vorliegen bzw. wonach erfindungsgemäß eine Hyaluronsäure bzw. ein hochmolekulares Hyaluronsäure-Salz eingesetzt wird, eine maßgebliche Bedeutung im Hinblick auf die überraschend gefundene Stabilisierung bzw. Wirksamkeitssteigerung der erfindungsgemäßen Zusammensetzung zu:
Denn durch den zweckgerichteten Einsatz einer hochmolekularen Hyaluronsäure bzw. eines hochmolekularen Hyaluronsäure-Salzes werden, ohne sich auf diese Theorie beschränken bzw. festlegen zu wollen, in der erfindungsgemäßen Zusammensetzung übergeordnete bzw. komplexe dreidimensionale Strukturen geschaffen, wobei in diesem Zusammenhang die Hyaluronsäure bzw. das Hyaluronsäure-Salz insbesondere in einer mizellenartigen Anordnung bzw. in Form einer hydrogelartigen Struktur vorliegt bzw. eine solche Anordnung / Struktur auszubilden imstande ist, und zwar insbesondere auch infolge einer Wechselwirkung der Hyaluronsäure bzw. des Hyaluronsäure-Salzes mit in der Zusammensetzung vorliegendem Wasser bzw. infolge einer Bindung von Wasser an die Hyaluronsäure bzw. das Hyaluronsäure-Salz. Hierbei können beispielsweise entropiegetriebene Prozesse, hydrophile Wechselwirkungen bzw. Dipol/Dipol-Wechselwirkungen und/oder Van-der-Waals-Wechselwirkungen eine gewisse Rolle spielen.

Folglich wird eine definierte dreidimensionale Matrix auf Basis der Hyaluronsäure bzw. des Hyaluronsäure-Salzes in der Zusammensetzung bereitgestellt, in welche die entsprechenden Wirk- bzw. Inhaltsstoffe, insbesondere die Komponenten (a) und (b), gewissermaßen eingelagert bzw. hiervon umgeben bzw. umschlossen werden. Durch die in Rede stehende "Einlagerung" bzw. "Umschließung" der Wirk- bzw. Inhaltsstoffe, insbesondere der Komponenten (a) und (b), werden diese sozusagen abgeschirmt und vor einem unerwünschten Abbau geschützt, was zu der erfindungsgemäß aufgefundenen Stabilisierung der Zusammensetzung führt.

Gleichermaßen kann eine weiterführende Stabilisierung der in der Zusammensetzung vorliegenden Wirk- bzw. Inhaltsstoffe auch dadurch gewährleistet werden, dass - gleichermaßen ohne sich auf diese Theorie beschränken bzw. festlegen zu wollen - eine direkte Wechselwirkung der hochmolekularen Hyaluronsäure bzw. des Hyaluronsäure-Salzes einerseits mit den Wirk- bzw. Inhaltsstoffen, insbesondere den Komponenten (a) und (b), andererseits in der Zusammensetzung vorliegt, wobei hierfür intermolekulare Wechselwirkungen, z. B. gleichermaßen auf Basis von hydrophilen Wechselwirkungen bzw. Dipol/Dipol- oder Van-der-Waals-Wechselwirkungen, verantwortlich sein können. Insbesondere kann eine direkte Wechselwirkung der Hyaluronsäure bzw. des Hyaluronsäure-Salzes einerseits mit dem alpha-Sympathomimetikum auf Basis von Xylometazolin bzw. mit dem Pantothenol andererseits vorliegen, was sozusagen zu einer weiterführenden "Anbindung" der jeweiligen Wirk- bzw. Inhaltsstoffe an die Hyaluronsäure bzw. dem Hyaluronsäure-Salz mit einer damit einhergehenden Stabilisierung führt.

Durch die spezielle Ausgestaltung der erfindungsgemäß eingesetzten Hyaluronsäure bzw. des diesbezüglichen Hyaluronsäure-Salzes wird somit einer Zersetzung bzw. einem Abbau des alpha-Sympathomimetikums gemäß Komponente (a) sowie von Pantothenol gemäß Komponente (b) nachhaltig entgegengewirkt.

Zudem kann in der erfindungsgemäßen Zusammensetzung eine darüberhinausgehende Interaktion der Hyaluronsäure bzw. des Hyaluronsäure-Salzes in grundsätzlich vergleichbarer Art auch mit weiteren Stoffen, wie etwaigen Abbauprodukten, vorliegen, so dass diese Abbauprodukte entsprechend unschädlich gemacht bzw. "eingefangen" werden.

Weiterhin liegt in Bezug auf die erfindungsgemäße Zusammensetzung nicht zuletzt aufgrund der speziellen Stabilisierung der erfindungsgemäßen Zusammensetzung gleichermaßen ein geringeres Keimwachstum vor, was auch dazu führt, dass die erfindungsgemäßen Zusammensetzung, wie nachfolgend noch angeführt, frei von Konservierungsmitteln bzw. frei von Desinfektionsmitteln ausgebildet sein kann.

Die im Rahmen der vorliegenden Erfindung mit der hochmolekularen Hyaluronsäure einhergehende Stabilisierung der erfindungsgemäßen Zusammensetzung kann durch die gezielte Einstellung bzw. Vorgabe eines pH-Wert-Regimes, insbesondere unter Verwendung eines definierten Puffersystems, weiterführend erhöht werden. Folglich kann auch infolge der gezielten Einstellung eines pH-Wertes trotz der bevorzugten konservierungsmittelfreien bzw. desinfektionsmittelfreien Ausbildung der Zusammensetzung nach der Erfindung eine weiterführend verbesserte Lagerstabilität erreicht werden.

Was darüber hinaus die hohe Wirkeffizienz bzw. Wirksamkeit sowie die insgesamt verbesserte Verträglichkeit der erfindungsgemäßen Zusammensetzung anbelangt, so kommt auch diesbezüglich der erfindungsgemäß eingesetzten Hyaluronsäure bzw. dem Hyaluronsäure-Salz mit dem definierten Molekulargewicht eine entscheidende Bedeutung zu: Denn - ohne sich auf diese Theorie beschränken oder festlegen zu wollen - insbesondere infolge der speziellen Matrix- bzw. Hydrogelausbildung mit der Einlagerung der Wirk- bzw. Inhaltsstoffe, insbesondere der Komponenten (a) bzw. (b), resultiert ein gewisser *Controlled-release*-Effekt und somit eine kontrollierte Freisetzung bzw. Abgabe der Wirk- bzw. Inhaltsstoffe, was auch zu einer Vergleichmäßigung und zeitlichen Verlängerung der Wirkstofffreisetzung im Anwendungsfall führt.

Dabei ist auch beachtlich, dass die erfindungsgemäße Zusammensetzung infolge der speziellen Hyaluronsäure bzw. des speziellen Hyaluronsäure-Salzes bei deren Anwendung bzw. Applikation verbesserte bzw. definierte mucoadhäsive Eigenschaften an der Nasenschleimhaut aufweist, so dass auch auf dieser Basis eine verlängerte Wirkdauer bzw. eine erhöhte Wirkeffizienz erreicht wird (längere Kontakt- bzw. Einwirkzeit der Zusammensetzung am Wirkort).

Aufgrund der vorgenannten Eigenschaften kann es erfindungsgemäß sogar vorgesehen sein, die Menge bzw. Dosis des alpha-Sympathomimetikums gegenüber einer hyaluronsäurefreien Applikationsform nachhaltig zu reduzieren, was auch zu einer verbesserten Verträglichkeit bzw. zu verringerten Nebenwirkungen führt.

Darüber hinaus kann die spezielle Hyaluronsäure bzw. das diesbezügliche Hyaluronsäure-Salz auch zu einer verbesserten Befeuchtung der Nasenschleimhaut führen, so dass auch infolgedessen einer Rhinitis bzw. den damit einhergehenden Symptomen effektiv entgegengewirkt werden kann. Insbesondere kann durch die hochmolekulare Hyaluronsäure bzw. das hochmolekulare Hyaluronsäure-Salz die Reepithelialisierung (Reepithelisation) des bei Rhinitis vorliegenden wunden bzw. geschädigten Nasenepithels gefördert werden. Dabei kann der erfindungsgemäßen Zusammensetzung bei deren topischen, insbesondere nasalen Anwendung sozusagen die zusätzliche Funktion eines "Feuchtepflasters" zukommen.

Gleichermaßen kann - ohne sich auf diese Theorie beschränken bzw. festlegen zu wollen - der Kombination von Dexpanthenol einerseits und der speziellen Hyaluronsäure bzw. des diesbezüglichen Hyaluronsäure-Salzes andererseits auch gewisser Enhancer-Effekt zugesprochen werden, so dass hierdurch eine Resorptionsverstärkung sowie eine verlängerte Verweilzeit der Wirksubstanzen bei entsprechender Applikation gewährleistet ist.

Aufgrund der erfindungsgemäß vorliegenden verbesserten Mucoadhäsivitität bzw. der definierten Viskosität der erfindungsgemäßen Zusammensetzung werden bei der Anwendung bzw. Applikation, sofern überhaupt, geringere Mengen der Zusammensetzung durch den Patienten verschluckt, was zu einer Verringerung der systemischen Resorption, einhergehend mit verringerten systemischen Nebenwirkungen, insbesondere infolge der systemischen Aufnahme des alpha-Sympathomimetikums, führt.

Aufgrund der verbesserten Mucoadhäsivitität sind insbesondere auch die organoleptischen Eigenschaften verbessert, insbesondere was den gegebenenfalls vorliegenden bitteren Geschmack der zugrundeliegenden Zusammensetzung anbelangt.

Aufgrund des hohen Molekulargewichts der erfindungsgemäß eingesetzten Hyaluronsäure bzw. des eingesetzten Hyaluronsäure-Salzes kann zudem deren bzw. dessen Menge bzw. Gehalt in der erfindungsgemäßen Zusammensetzung verringert werden, so dass insgesamt nur relativ geringe Mengen an Hyaluronsäure bzw. Hyaluronsäure-Salz in die Zusammensetzung nach der Erfindung inkorporiert werden müssen, was gleichermaßen auch der in Rede stehenden Verkeimung der Zusammensetzung entgegenwirkt, da - ohne sich auf diese Theorie beschränken bzw. festlegen zu wollen - höhere Mengen bzw. Gehalte an Hyaluronsäure in der Zusammensetzung nach der Erfindung andernfalls als Nährboden für Keime dienen könnten.

Erfindungsgemäß geht somit die Verwendung einer sehr speziellen Hyaluronsäure bzw. eines speziellen Hyaluronsäure-Salzes gemäß Komponente (c) in Kombination mit dem in Rede stehenden alpha-Sympathomimetikum gemäß Komponente (a) und Pantothenol gemäß Komponente (b) in einer gemeinsamen und vorzugsweis wässrig ausgebildeten Zusammensetzung mit zahlreichen anwendungsspezifischen und medizinisch-pharmakologischen Vorteilen einher. Dabei ist auch hervorzuheben, dass die zweckgerichtete Kombination der Komponenten (a), (b) und (c) aufgrund des speziellen Zusammenwirkens bzw. der speziellen Interaktion der jeweiligen Komponenten und der zugrundeliegenden multiplen Wirkeffekte zu über die Summe der Eigenschaften der Einzelkomponenten hinausgehenden Eigenschaften der Zusammensetzung nach der Erfindung führen, so dass diesbezüglich im Rahmen der vorliegenden Erfindung auch ein synergistischer Effekt vorliegt.

Erfindungsgemäß kann der Gehalt bzw. die Menge an Hyaluronsäure bzw. physiologisch verträglichem Hyaluronsäure-Salz in weiten Bereichen variieren. Erfindungsgemäß ist es jedoch bevorzugt, wenn die Zusammensetzung die Komponente (c) und/oder die Hyaluronsäure bzw. das physiologisch verträgliche Hyaluronsäure-Salz in einer Menge im Bereich von 0,0001 Gew.-% bis 5 Gew.-%, insbesondere im Bereich von 0,0005 Gew.-% bis 3 Gew.-%, vorzugsweise im Bereich von 0,001 Gew.-% bis 1 Gew.-%, bevorzugt im Bereich von 0,005 Gew.-% bis 0,5 Gew.-%, besonders bevorzugt im Bereich von 0,01 Gew.-% bis 0,1 Gew.-%, bezogen auf die Zusammensetzung, enthält. Wie zuvor angeführt, können im Rahmen der vorliegenden Erfindung insbesondere infolge des Einsatzes einer hochmolekularen Hyaluronsäure vergleichsweise geringe Mengen bzw. Gehalte an Hyaluronsäure realisiert werden, um die gewünschten Effekte, wie zuvor angeführt, zu gewährleisten.

Wie zuvor angeführt, kommt dem Molekulargewicht (synonym auch als "Molmasse" bezeichnet) der Komponente (c) eine hohe Bedeutung zu. Hierzu kann in Ergänzung zu den obigen Ausführungen auf Folgendes abgestellt werden:
Insbesondere kann es erfindungsgemäß vorgesehen sein, dass die Komponente (c) und/oder die Hyaluronsäure bzw. das physiologisch verträgliche Hyaluronsäure-Salz ein zahlenmittleres Molekulargewicht (Molmasse) Mₙ im Bereich von 0,6 MDa bis 4,5 MDa, insbesondere im Bereich von 0,7 MDa bis 4 MDa, vorzugsweise im Bereich von 0,75 MDa bis 3,5 MDa, bevorzugt im Bereich von 0,8 MDa bis 3,25 MDa, besonders bevorzugt im Bereich von 0,9 MDa bis 3 MDa, aufweist.

Insbesondere kann die Komponente (c) und/oder die Hyaluronsäure bzw. das physiologisch verträgliche Hyaluronsäure-Salz ein gewichtsmittleres Molekulargewicht (Molmasse) M_{w} im Bereich von 0,7 MDa bis 5,5 MDa, insbesondere im Bereich von 0,8 MDa bis 5 MDa, vorzugsweise im Bereich von 0,85 MDa bis 4,5 MDa, bevorzugt im Bereich von 0,9 MDa bis 4 MDa, besonders bevorzugt im Bereich von 0,95 MDa bis 3 MDa, aufweisen.

Zudem kann die Komponente (c) und/oder die Hyaluronsäure bzw. das physiologisch verträgliche Hyaluronsäure-Salz ein zentrifugenmittleres Molekulargewicht (Molmasse) M_{z} im Bereich von 0,7 MDa bis 7 MDa, insbesondere im Bereich von 0,8 MDa bis 6 MDa, vorzugsweise im Bereich von 0,9 MDa bis 5 MDa, bevorzugt im Bereich von 0,95 MDa bis 4 MDa, besonders bevorzugt im Bereich von 1 MDa bis 3,5 MDa, ganz besonders bevorzugt im Bereich von 1,1 MDa bis 3 MDa, aufweisen.

In diesem Zusammenhang kommt auch dem Polydispersitätsindex (PDI) eine entsprechende Bedeutung zu: Diesbezüglich kann es erfindungsgemäß vorgesehen sein, dass die Komponente (c) und/oder die Hyaluronsäure bzw. das physiologisch verträgliche Hyaluronsäure-Salz einen Polydispersitätsindex (PDI), berechnet als Quotient aus gewichtsmittlerem Molekulargewicht M_{w} und zahlenmittlerem Molekulargewicht Mₙ [M_{w}/Mₙ], von mindestens 0,9, insbesondere mindestens 0,95, vorzugsweise mindestens 1, bevorzugt mindestens 1,05, besonders bevorzugt mindestens 1,1, aufweist.

Insbesondere kann die Komponente (c) und/oder die Hyaluronsäure bzw. das physiologisch verträgliche Hyaluronsäure-Salz einen Polydispersitätsindex (PDI), berechnet als Quotient aus gewichtsmittlerem Molekulargewicht M_{w} und zahlenmittlerem Molekulargewicht Mₙ [M_{w}/Mₙ], von höchstens 10, insbesondere höchstens 5, vorzugsweise höchstens 3, bevorzugt höchstens 2, besonders bevorzugt höchstens 1,5, aufweisen.

Gleichermaßen kann die Komponente (c) und/oder die Hyaluronsäure bzw. das physiologisch verträgliche Hyaluronsäure-Salz einen Polydispersitätsindex (PDI), berechnet als Quotient aus gewichtsmittlerem Molekulargewicht M_{w} und zahlenmittlerem Molekulargewicht Mₙ [M_{w}/Mₙ], im Bereich von 0,9 bis 10, insbesondere im Bereich von 0,95 bis 5, vorzugsweise im Bereich von 1 bis 3, bevorzugt im Bereich von 1,05 bis 2, besonders bevorzugt im Bereich von 1,1 bis 1,5, aufweisen.

Die vorgenannten Eigenschaften der Komponente (c) hinsichtlich des Molekulargewichts bzw. der Molmasse bzw. des diesbezüglichen Polydispersitätsindexes führen - ohne sich auf diese Theorie beschränken oder festlegen zu wollen - zu einer weiterführend verbesserten Ausbildung der mizellenartigen Strukturen bzw. der der Zusammensetzung nach der Erfindung zugrundeliegenden Matrix bzw. des durch die Hyaluronsäure bzw. das diesbezügliche Hyaluronsäure-Salz unter Einbezug von Wasser ausgebildeten Hydrogels mit entsprechender Stabilisierung der jeweiligen Wirk- bzw. Inhaltsstoffe und mit entsprechender Erhöhung der Wirkeffizienz der erfindungsgemäßen Zusammensetzung, und zwar insbesondere auch im Hinblick auf den vorgenannten mucoadhäsiven Effekt bei topischer bzw. intranasaler Anwendung bzw. Applikation der erfindungsgemäßen Zusammensetzung.

Die vorgenannten Molekulargewichte können dabei mit dem Fachmann an sich bekannten Methoden bestimmt werden.

Erfindungsgemäß kann das zahlenmittlere Molekulargewicht Mₙ und/oder das gewichtsmittlere Molekulargewicht M_{w} und/oder das zentrifugenmittlere Molekulargewicht M_{z} und/oder der Polydispersitätsindex (PDI) der Komponente (c) und/oder der Hyaluronsäure bzw. des physiologisch verträglichen Hyaluronsäure-Salzes mittels Gelpermeationschromatographie (GPC), insbesondere gekoppelt mit Lichtstreudetektion (MALLS; *Multi Angle Laser Light Scattering*), und/oder gemäß DIN 55672-3:2016-03 bestimmt sein.

Zudem kann das zahlenmittlere Molekulargewicht Mₙ bzw. das gewichtsmittlere Molekulargewicht M_{w} bzw. das zentrifugenmittlere Molekulargewicht M_{z} bzw. der Polydispersitätsindex (PDI) der Komponente (c) und/oder der Hyaluronsäure bzw. des physiologisch verträglichen Hyaluronsäure-Salzes mittels Gelpermeationschromatographie (GPC), insbesondere gekoppelt mit Lichtstreudetektion (MALLS), insbesondere bei einer Temperatur im Bereich von 20 °C bis 40 °C und/oder mit einer phosphatgepufferten (PBS-Puffer; *phosphate buffered saline*) 0,01 mol/l NaCI-Lösung mit einem pH-Wert von 7,4 als Elutionsmittel und/oder an einer Lösung von Hyaluronsäure und/oder des physiologisch verträglichen Hyaluronsäure-Salzes mit einer Konzentration von 2,565 g/l und/oder unter Verwendung eines Dextran/Pullulan-Standards als Kalibrierreagenz und/oder gemäß DIN 55672-3:2016-03, bestimmt sein.

Erfindungsgemäß kann es zudem vorgesehen sein, dass die Hyaluronsäure bzw. das physiologisch verträgliche Hyaluronsäure-Salz (Komponente (c)) nichttierischen Ursprungs ist.

In diesem Zusammenhang kann die Hyaluronsäure bzw. das physiologisch verträgliche Hyaluronsäure-Salz (Komponente (c)) bakteriellen bzw. fermentativen Ursprungs, insbesondere fermentativen Ursprungs, sein.

Diesbezüglich kann die Hyaluronsäure bzw. das physiologisch verträgliche Hyaluronsäure-Salz (Komponente (c)) vorzugsweise fermentativ von Bakterien der Gattung *Streptococcus,* insbesondere *Streptococcus lancefields,* bevorzugt *Streptococcus lancefields* Stamm A, gewonnen sein.

In erfindungsgemäß bevorzugter Weise kann somit die Hyaluronsäure und/oder das physiologisch verträgliche Hyaluronsäure-Salz (Komponente (c)) in Form einer bakteriell und/oder fermentativ, insbesondere fermentativ gewonnenen Hyaluronsäure und/oder in Form eines bakteriell bzw. fermentativ, insbesondere fermentativ gewonnenen Hyaluronsäure-Salzes (Komponente (c)) eingesetzt sein.

In diesem Zusammenhang hat die Anmelderin gleichermaßen in überraschender Weise herausgefunden, dass durch die Verwendung einer nichttierischen Hyaluronsäure der vorgenannten Art auch die pharmazeutische bzw. pharmakologische Wirksamkeit der erfindungsgemäßen Zusammensetzung in Bezug auf die Behandlung von Rhinitiden, insbesondere *Rhinitis acuta,* signifikant verbessert ist. Ohne sich auf diese spezielle Theorie beschränken bzw. festlegen zu wollen, kann dies damit begründet werden, dass es sich bei der nichttierischen, insbesondere bakteriell gewonnenen Hyaluronsäure um ein besonders reines Produkt mit definierten chemischen bzw. physikalischen Eigenschaften handelt, welches gleichermaßen hochwirksam ist. Die definierte Ausbildung der Hyaluronsäure mit der hohen Reinheit ist gleichermaßen auch der Stabilität der Zusammensetzung nach der Erfindung weiterführend zuträglich, da keine der Stabilität abträglichen Verunreinigungen zugegen sind. Zudem ist die nichttierische Hyaluronsäure gut verträglich, da eine Kontamination bzw. Verunreinigung mit anderen Stoffen, wie es oftmals bei tierisch gewonnenen Produkten der Fall ist, nicht vorliegt. Somit weist Hyaluronsäure nichttierischen Ursprungs eine besonders hohe Reinheit und Homogenität auf, was auch der Stabilität, insbesondere Langzeitstabilität, der erfindungsgemäßen Zusammensetzung zuträglich ist.

Gemäß einer erfindungsgemäß bevorzugten Ausführungsform kann die Hyaluronsäure bzw. das physiologisch verträgliche Hyaluronsäure-Salz (Komponente (c)) in Form eines Alkalisalzes, vorzugsweise in Form eines Natrium-Salzes, und/oder in Form eines Alkalihyaluronates vorliegen. Dabei werden gleichermaßen besonders gute Ergebnisse erhalten, wenn die Hyaluronsäure bzw. das physiologisch verträgliche Hyaluronsäure-Salz (Komponente (c)) in Form von Natriumhyaluronat vorliegt.

Die Komponente (c) der erfindungsgemäßen Zusammensetzung kann insbesondere auf Basis einer sterilen bzw. hochgereinigten Lösung oder Suspension insbesondere den Natriumsalzes der Hyaluronsäure eingesetzt werden.

Im Rahmen der vorliegenden Erfindung einsetzbare Hyaluronsäure bzw. einsetzbares Hyaluronsäure-Salz ist im Allgemeinen kommerziell erhältlich, beispielsweise von GFN Herstellung von Naturextrakten GmbH, Wald-Michelbach (DE), Contipro S. A., Dolni Dobrouc (C. Z.) oder Vivatis Pharma GmbH, Hamburg (DE).

Für weitergehende Einzelheiten zum Begriff der Hyaluronsäure bzw. deren physiologisch verträglichen Salze kann auf RÖMPP Chemielexikon, 10. Auflage, Band 3, 1997, Georg-Thieme-Verlag Stuttgart/New York, Seite 1820, Stichwort: "Hyaluronsäure", sowie auf die dort referierte Literatur verwiesen werden, wobei der Gesamtoffenbarungsgehalt der vorgenannten Literatur hiermit durch Bezugnahme vollumfänglich eingeschlossen ist.

Darüber hinaus ist es erfindungsgemäß vorgesehen, dass die Komponente (a) und/oder das imidazolinbasierte alpha-Sympathomimetikum bzw. dessen physiologisch verträgliches Salz ausgewählt ist aus Xylometazolin, insbesondere in Form von dessen physiologisch unbedenklichen Salzen, besonders bevorzugt in Form von dessen Hydrochloridsalz (Xylometazolinhydrochlorid). Erfindungsgemäß ist es somit vorgesehen, dass die Komponente (a) Xylometazolin, insbesondere in Form von dessen physiologisch unbedenklichen Salzen, besonders bevorzugt in Form von dessen Hydrochloridsalz (Xylometazolinhydrochlorid), ist. In besonders bevorzugter Weise ist die Komponente (a) somit Xylometazolinhydrochlorid.

Was weiterhin die in der erfindungsgemäßen Zusammensetzung vorgesehene Komponente (b) anbelangt, so kann die Komponente (b) aus Pantothenol (Dexpanthenol) oder dessen physiologisch unbedenklichen Estern ausgewählt sein. Vorzugsweise ist die Komponente (b) Pantothenol (Dexpanthenol).

Auch kommt den jeweiligen Mengen bzw. Gehalten an Komponente (a) bzw. Komponente (b) in der erfindungsgemäßen Zusammensetzung eine große Bedeutung zu. Diesbezüglich können die jeweiligen Mengen in weiten Bereichen variieren, wobei jedoch die nachfolgend angeführten Mengen bzw. Gehalte zu besonders guten Ergebnissen führen:
So kann die Zusammensetzung die Komponente (a) und/oder das Xylometazolin bzw. dessen physiologisch verträgliches Salz in einer Menge im Bereich von 0,001 bis 2 Gew.-%, insbesondere im Bereich von 0,005 Gew.-% bis 1,5 Gew.-%, vorzugsweise im Bereich von 0,01 Gew.-% bis 1,2 Gew.-%, bevorzugt im Bereich von 0,02 Gew.-% bis 1,0 Gew.-%, besonders bevorzugt im Bereich von 0,03 Gew.-% bis 0,5 Gew.-%, ganz besonders bevorzugt im Bereich von 0,04 Gew.-% bis 0,2 Gew.-%, noch weiter bevorzugt im Bereich von 0,045 Gew.-% bis 0,175 Gew.-%, bezogen auf die Zusammensetzung, enthalten.

Zudem kann die Zusammensetzung nach der Erfindung die Komponente (b) und/oder Pantothenol oder dessen physiologisch unbedenkliche Ester in einer Menge im Bereich von 0,01 Gew.-% bis 10 Gew.-%, insbesondere im Bereich von 0,1 Gew.-% bis 9 Gew.-%, vorzugsweise im Bereich von 0,5 Gew.-% bis 8 Gew.-%, bevorzugt im Bereich von 1 Gew.-% bis 7 Gew.-%, besonders bevorzugt im Bereich von 2 Gew.-% bis 6 Gew.-%, ganz besonders bevorzugt im Bereich von 3 Gew.-% bis 6 Gew.-%, weiter bevorzugt im Bereich von 3,5 Gew.-% bis 5,5 Gew.-%, bezogen auf die Zusammensetzung, enthalten.

Zudem kann es erfindungsgemäß vorgesehen sein, dass die Zusammensetzung die Komponenten (a) und (b) in einem Mengenverhältnis von Komponente (a) zu Komponente (b) [Komponente (a) : Komponente (b)] im Bereich von 1 : 10 bis 1 : 1.000, insbesondere im Bereich von 1 : 15 bis 1 : 500, vorzugsweise im Bereich von 1 : 20 bis 1 : 250, bevorzugt im Bereich von 1 : 25 bis 1 : 200, besonders bevorzugt im Bereich von 1 : 30 bis 1 : 175, ganz besonders bevorzugt im Bereich von 1 : 40 bis 1 : 150, weiter bevorzugt im Bereich von 1 : 45 bis 1 : 125, enthält.

Weiterhin kann die Zusammensetzung die Komponenten (a) und (c) in einem Mengenverhältnis von Komponente (a) zu Komponente (c) [Komponente (a) : Komponente (c)] im Bereich von 1 : 10 bis 100 : 1, insbesondere im Bereich von 1 : 5 bis 50 : 1, vorzugsweise im Bereich von 1 : 2 bis 30 : 1, bevorzugt im Bereich von 1 : 1 bis 20 : 1, besonders bevorzugt im Bereich von 2 : 1 bis 10 : 1, enthalten.

Darüber hinaus kann es erfindungsgemäß vorgesehen sein, dass die Zusammensetzung die Komponenten (b) und (c) in einem Mengenverhältnis von Komponente (b) zu Komponente (c) [Komponente (b) : Komponente (c)] im Bereich von 1 : 2 bis 5.000 : 1, insbesondere im Bereich von 1 : 1 bis 2.500 : 1, vorzugsweise im Bereich von 2 : 1 bis 1.000 : 1, bevorzugt im Bereich von 5 : 1 bis 750 : 1, besonders bevorzugt im Bereich von 10 : 1 bis 500 : 1, enthält.

Im Allgemeinen liegt die erfindungsgemäße Zusammensetzung als wässrige Zusammensetzung vor. Insbesondere ist also die erfindungsgemäße Zusammensetzung wässrig basiert bzw. liegt die erfindungsgemäße Zusammensetzung wässrig formuliert vor, insbesondere in Form einer wässrigen Lösung oder wässrigen Solubilisierung. Insbesondere ist auf diese Weise eine optimale Ausbildung der in Rede stehenden Matrix bzw. des zugrundeliegenden Hydrogels auf Basis der Hyaluronsäure bzw. des diesbezüglichen Hyaluronsäure-Salzes gewährleistet.

In bevorzugter Weise liegt somit die erfindungsgemäße Zusammensetzung als wässriges System, insbesondere als wässriges Einphasensystem, vorzugsweise als wässrige Lösung oder wässrige Solubilisierung, vor. Insbesondere weist die erfindungsgemäße Zusammensetzung somit einen Exzipienten oder Träger auf Wasserbasis und/oder in Form von Wasser, insbesondere gereinigtem Wasser, auf.

Im Allgemeinen liegt die erfindungsgemäße Zusammensetzung als zumindest im Wesentlichen klare, farblose wässrige Lösung vor. Dies hat zum einen den Vorteil einer vereinfachten Applikation. Zum anderen lassen sich an einer klaren, farblosen wässrigen Lösung etwaige Veränderungen ohne Weiteres wahrnehmen.

Was die eingesetzte Menge des Exzipienten bzw. Trägers auf Wasserbasis und/oder in Form von Wasser bzw. die eingesetzte Menge an Wasser anbelangt, so kann diese in weiten Bereichen variieren. Erfindungsgemäß ist es bevorzugt, wenn die Zusammensetzung nach der Erfindung einen diesbezüglichen Gehalt, insbesondere einen Gehalt an Wasser, insbesondere an gereinigtem Wasser, von mindestens 50 Gew.-%, insbesondere mindestens 75 Gew.-%, vorzugsweise 80 Gew.-%, bevorzugt mindestens 85 Gew.-%, besonders bevorzugt mindestens 90 Gew.-%, bezogen auf die Zusammensetzung, aufweist.

Was die erfindungsgemäße Zusammensetzung in diesem Zusammenhang weiterhin anbelangt, so liegen die angeführten Wirk- bzw. Inhaltsstoffe, insbesondere die Komponenten (a), (b) und (c) sowie gegebenenfalls weitere vorhandene Inhaltsstoffe, somit in gemeinsamer wässriger Lösung bzw. Solubilisierung, vorzugsweise in gemeinsamer wässriger Lösung, vor. Aufgrund der erfindungsgemäßen Konzeption ist dabei, wie zuvor angeführt, eine effektive Stabilisierung der in Rede stehenden Wirk- bzw. Inhaltsstoffe gewährleistet.

Nicht zuletzt aufgrund der hohen Stabilität, insbesondere Lagerstabilität, der erfindungsgemäßen Zusammensetzung kann es im Rahmen der vorliegenden Erfindung gemäß einer bevorzugten Ausführungsform vorgesehen sein, dass die Zusammensetzung keine Konservierungsmittel bzw. keine Desinfektionsmittel aufweist. Insbesondere kann die Zusammensetzung nach der Erfindung frei von Konservierungsmitteln und/oder frei von Desinfektionsmitteln sein. Diesbezüglich kann es erfindungsgemäß somit insbesondere vorgesehen sein, dass die Zusammensetzung konservierungsmittelfrei und/oder desinfektionsmittelfrei ausgebildet ist.

Hierdurch wird die Verträglichkeit der erfindungsgemäßen Zusammensetzung nochmals verbessert, da erfindungsgemäß auf den Einsatz von auf die Nasenschleimhaut gegebenenfalls reizend wirkenden Zusatzstoffen verzichtet werden kann.

Im Rahmen der vorliegenden Erfindung hat die Anmelderin zudem in völlig überraschender Weise gefunden, dass eine weiterführende Stabilisierung der erfindungsgemäßen Zusammensetzung durch Einstellung eines definierten pH-Werts erreicht werden kann. Hierdurch kann die Stabilisierung der erfindungsgemäßen Zusammensetzung, insbesondere der Wirkstoffe (a) und (b) bzw. der Komponenten (a) und (b) der erfindungsgemäßen Zusammensetzung, weiterführend verbessert werden.

Erfindungsgemäß ist es bevorzugt, wenn der pH-Wert der Zusammensetzung im Bereich von 4,9 bis 6,1, insbesondere im Bereich von 5,0 bis 6,0, vorzugsweise im Bereich von 5,1 bis 6,0, bevorzugt im Bereich von 5,2 bis 5,9, besonders bevorzugt im Bereich von 5,25 bis 5,85, eingestellt bzw. konstantgehalten wird. Aufgrund der vorgenannten Eigenschaften im Hinblick auf den pH-Wert ist die erfindungsgemäße Zusammensetzung zudem besonders gut verträglich.

Im Rahmen der vorliegenden Erfindung kann die Bestimmung des pH-Wertes mit dem Fachmann an sich bekannten Methoden erfolgen. Insbesondere kann die Bestimmung des pH-Wertes auf Basis einer potentiometrischen Analyse erfolgen. Vorzugsweise kann die Bestimmung des pH-Wertes gemäß der Methode nach Ph. Eur. [Pharmacopoea Europaea], 9th Edition (9.0), 2017, 9. Auflage Englisch, Abschnitt 2.2.3. "Potentiometric determination of pH" erfolgen.

Für weitergehende Einzelheiten zum Begriff des pH-Wertes kann insbesondere verwiesen werden auf RÖMPP Lexikon Chemie, 10. Auflage, Georg-Thieme-Verlag, Stuttgart/New York, Band 4, 1998, Seiten 3230 bis 3232, Stichwort: "pH", sowie auf die dort referierte Literatur, wobei der gesamte Offenbarungsgehalt der vorgenannten Literatur hiermit durch Bezugnahme vollumfänglich eingeschlossen ist.

Üblicherweise erfolgt die Einstellung und/oder Konstanthaltung des pH-Werts der Zusammensetzung nach der Erfindung mittels mindestens eines chemischen Puffersystems, insbesondere Puffersalz(en) (Komponente (d)). Mit anderen Worten kann die erfindungsgemäße Zusammensetzung somit mindestens ein chemisches Puffersystem, insbesondere Puffersalz(en), enthalten.

Zum Begriff des chemischen Puffers bzw. Puffersystems kann insbesondere verwiesen werden auf RÖMPP Lexikon Chemie, 10. Auflage, Georg-Thieme-Verlag, Stuttgart/New York, Band 5, 1998, Seiten 3618/3619, Stichwort: "Puffer", sowie auf die dort referierte Literatur, wobei die gesamte vorgenannte Literaturstelle mit der dort referierten Literatur hiermit durch Bezugnahme eingeschlossen ist.

Im Allgemeinen kann die Zusammensetzung nach der Erfindung das chemische Puffersystem (Komponente (d)) in einer Menge im Bereich von 0,01 Gew.-% bis 5 Gew.-%, vorzugsweise im Bereich von 0,05 Gew.-% bis 4 Gew.-%, bevorzugt im Bereich von 0,1 Gew.-% bis 3 Gew.-%, besonders bevorzugt im Bereich von 0,5 Gew.-% bis 2 Gew.-%, bezogen auf die Zusammensetzung, enthalten.

In diesem Zusammenhang hat es sich insbesondere bewährt, wenn als chemisches Puffersystem (Komponente (d)) bevorzugt ein Dihydrogenphosphat/Monohydrogenposphat-Puffersystem ("H₂PO₄⁻/HPO₄²⁻-Puffer(system)" bzw. "Phosphatpuffer(system)"), insbesondere ein Alkalidihydrogenphosphat/Alkalimonohydrogenposphat-Puffersystem, eingesetzt wird.

Insbesondere kann die Zusammensetzung somit als chemisches Puffersystem (Komponente (d)) bevorzugt ein Dihydrogenphosphat/Monohydrogenposphat-Puffersystem ("H₂PO₄⁻/HPO₄²⁻-Puffer(system)" bzw. "Phosphatpuffer(system)"), insbesondere ein Alkalidihydrogenphosphat/Alkalimonohydrogenposphat-Puffersystem, enthalten. Insbesondere kann ein Kaliumdihydrogenphosphat/Natriummonohydrogenposphat-Puffersystem zum Einsatz kommen.

Denn die Anmelderin hat in diesem Zusammenhang gleichermaßen völlig überraschend gefunden, dass infolge der Verwendung des zuvor genannten speziellen Puffersystems die Stabilität der erfindungsgemäßen Zusammensetzung nochmals weiterführend verbessert ist. Somit zeichnet sich das erfindungsgemäß in bevorzugter Weise eingesetzte Phosphatpuffersystem - gegenüber anderen möglichen einsetzbaren Puffersystemen - dadurch aus, dass auf dieser Basis eine weiterführende Verbesserung der Langzeitstabilisierung der erfindungsgemäßen Zusammensetzung erreicht werden kann. Mit anderen Puffersystemen dagegen, welche sich im vergleichbaren pH-Wertebereich einsetzen lassen, wie z. B. einem Kohlensäure/Bicarbonat-Puffersystem, einem Essigsäure/Acetat-Puffersystem, einem Kohlensäure/Silikat-Puffersystem, einem Citronensäure/Citrat-Puffersystem oder dergleichen, lassen sich derart gute Stabilitätseigenschaften nicht bzw. nicht immer zuverlässig erhalten. Ohne sich auf eine bestimmte Theorie beschränken oder festlegen zu wollen, lässt sich dieser Effekt des erfindungsgemäß bevorzugt eingesetzten Phosphatpuffersystems möglicherweise auf sekundäre Effekte, wie Komplexierungsreaktionen oder dergleichen, zurückführen.

Insbesondere kann es erfindungsgemäß vorgesehen sein, dass das Dihydrogenphosphat/Monohydrogenposphat-Puffersystem mit einem Dihydrogenphosphat/Monohydrogenposphat-Molverhältnis von größer 5 : 1, insbesondere im Bereich von 5 : 1 bis 110 : 1, besonders bevorzugt im Bereich von 6 : 1 bis 105 : 1, besonders bevorzugt im Bereich von 7 : 1 bis 100 : 1, ganz besonders bevorzugt im Bereich von 8 : 1 bis 90 : 1, eingesetzt wird.

Mit anderen Worten kann es erfindungsgemäß gleichermaßen vorgesehen sein, dass die Zusammensetzung das Dihydrogenphosphat/Monohydrogenposphat-Puffersystem mit einem Dihydrogenphosphat/Monohydrogenposphat-Molverhältnis von größer 5 : 1, insbesondere im Bereich von 5 : 1 bis 110 : 1, besonders bevorzugt im Bereich von 6 : 1 bis 105 : 1, besonders bevorzugt im Bereich von 7 : 1 bis 100 : 1, ganz besonders bevorzugt im Bereich von 8 : 1 bis 90 : 1, enthält.

Im Rahmen der vorliegenden Erfindung kann es insbesondere vorgesehen sein, dass die Zusammensetzung nach der Erfindung zumindest im Wesentlichen aus den zuvor angeführten Bestandteilen in Form der Komponente (a), der Komponente (b), der Komponente (c) und der Komponente (d) sowie Wasser als Exzipient bzw. Träger besteht. Demnach kann erfindungsgemäß eine Zusammensetzung bereitgestellt werden, welche zumindest im Wesentlichen aus den Komponenten (a), (b), (c) sowie (d) und Wasser besteht.

Im Rahmen der vorliegenden Erfindung kann es jedoch auch vorgesehen sein, dass die Zusammensetzung mindestens einen physiologisch verträglichen Elektrolyten (Komponente (e)) enthält. In diesem Zusammenhang kann der Elektrolyt in Form eines Alkalisalzes, insbesondere in Form eines Alkalichlorids, vorzugsweise in Form von Natriumchlorid, vorliegen bzw. ausgebildet sein. Gemäß einer erfindungsgemäß bevorzugten Ausführungsform kann der Elektrolyt (Komponente (e)) Natriumchlorid sein. Der in Rede stehende Elektrolyt bzw. Natriumchlorid kann im Rahmen der Verabreichung bzw. Applikation der erfindungsgemäßen Zusammensetzung eine weiterführende Befeuchtung der Nasenschleimhäute und somit eine Beschleunigung der Regeneration entzündeter Nasenschleimhäute gewährleisten, was gleichermaßen der Reepithelialisierung (Reepithelisation) und somit dem Heilungserfolg insgesamt zuträglich ist.

Erfindungsgemäß kann es dabei vorgesehen sein, dass die Zusammensetzung den Elektrolyten (Komponente (e)) in einer Menge im Bereich von 0,001 Gew.-% bis 5 Gew.-%, insbesondere im Bereich von 0,01 Gew.-% bis 2 Gew.-%, vorzugsweise im Bereich von 0,1 Gew.-% bis 1 Gew.-%, bezogen auf die Zusammensetzung, enthält.

Gleichermaßen kann es im Rahmen der vorliegenden Erfindung vorgesehen sein, dass die erfindungsgemäße Zusammensetzung mindestens einen weiteren Inhaltsstoff aufweist. Dieser weitere Inhaltsstoff kann insbesondere ausgewählt sein aus der Gruppe von Verarbeitungshilfsstoffen, Stabilisatoren, Emulgatoren, Antioxidantien, Feuchthaltemitteln, Verdickungsmitteln, Farbstoffen, Aromastoffen, Riechstoffen, Duftstoffen, Streckmitteln, Bindemitteln, Netzmitteln, Vitaminen, Spurenelementen, Mineralstoffen, Mikronährstoffen und/oder ätherischen Ölen sowie deren Kombinationen.

Weiterhin ist die Osmolalität der erfindungsgemäßen Zusammensetzung von Bedeutung. Die Osmolalität der erfindungsgemäßen Zusammensetzung kann dabei in weiten Bereichen variieren. Insbesondere um eine gute Verträglichkeit der erfindungsgemäßen Zusammensetzung in Bezug auf die Nasenschleimhaut zu gewährleisten, ist es von Vorteil, wenn die Zusammensetzung nach der Erfindung eine Osmolalität im Bereich von 275 mosmol/kg bis 625 mosmol/kg, insbesondere im Bereich von 300 mosmol/kg bis 600 mosmol/kg, vorzugsweise im Bereich von 310 mosmol/kg bis 550 mosmol/kg, bevorzugt im Bereich von 300 mosmol/kg bis 525 mosmol/kg, besonders bevorzugt im Bereich von 325 mosmol/kg bis 510 mosmol/kg, ganz besonders bevorzugt im Bereich von 340 mosmol/kg bis 505 mosmol/kg, weiter bevorzugt im Bereich von 350 mosmol/kg bis 500 mosmol/kg, noch weiter bevorzugt im Bereich von 350 mosmol/kg bis 495 mosmol/kg, aufweist. Auf diese Weise ist bei topischer Anwendung bzw. Verabreichung der erfindungsgemäßen Zusammensetzung auf die Nasenschleimhaut eine gute Verträglichkeit gewährleistet.

Die Osmolalität kann dabei gemäß dem Fachmann an sich bekannten Methoden bestimmt werden. Insbesondere kann die Osmolalität gemäß der Methode nach Ph. Eur. [Pharmacopoea Europaea], 9th Edition (9.0), 2017, 9. Auflage Englisch, Abschnitt 2.2.35. "Osmolality", bestimmt sein. Die Einstellung der Osmolalität kann beispielsweise auch auf Basis des zuvor genannten Elektrolyten (Komponente (d)), beispielsweise auf Basis von Alkali-Ionen, wie Natrium-Ionen, und Chlorid-Ionen, insbesondere mittels Natriumchlorid, erfolgen.

Darüber hinaus kann die Zusammensetzung nach der Erfindung bei einer Temperatur von 20 °C und bei einem Druck von 1.013,25 mbar eine relative Dichte, bezogen auf reines Wasser, im Bereich von 1,001 bis 1,2, insbesondere im Bereich von 1,005 bis 1,15, vorzugsweise im Bereich von 1,005 bis 1,105, aufweisen.

Im Rahmen der vorliegenden Erfindung kann die Bestimmung der Dichte mit dem Fachmann an sich bekannten Methoden erfolgen. Insbesondere kann die relative Dichte gemäß der Methode nach Ph. Eur. [Pharmacopoea Europaea], 9th Edition (9.0), 2017, 9. Auflage Englisch, Abschnitt 2.2.5. "Relative density" bestimmt sein. Durch die zweckgerichtete Einstellung der Dichte kann die Handhabung und Verträglichkeit der erfindungsgemäßen Zusammensetzung weiterführend verbessert werden.

Insbesondere kann die Zusammensetzung bei einer Temperatur von 20 °C eine dynamische Viskosität im Bereich von 0,5 mPas bis 100 mPas, insbesondere im Bereich von 0,75 mPas bis 50 mPas, vorzugsweise im Bereich von 1 mPas bis 10 mPas, bevorzugt im Bereich von 1,1 mPas bis 5 mPas, besonders bevorzugt im Bereich von 1,2 mPas bis 2,8 mPas, aufweisen.

Die Bestimmung der dynamischen Viskosität kann mit dem Fachmann an sich bekannten Methoden erfolgen. Insbesondere kann die dynamische Viskosität gemäß der Methode nach Ph. Eur. [Pharmacopoea Europaea], 9th Edition (9.0), 2017, 9. Auflage Englisch, Abschnitt 2.2.8. "Viscosity" und Abschnitt 2.2.9. "Capillary viscometer method" bestimmt sein.

Wie zuvor angeführt, weist die erfindungsgemäße Zusammensetzung eine hohe Stabilität, insbesondere Lagerstabilität, auf. Dabei kann die Stabilität, insbesondere Lagerstabilität, der erfindungsgemäßen Zusammensetzung durch das Vorhandensein bzw. den Gehalt der Abbauprodukte der Wirkstoffe bzw. Komponenten (a) und (b) bestimmt bzw. charakterisiert werden. Wie eingangs geschildert, ist das charakteristische Abbauprodukt von Xylometazolin bzw. Xylometazolinhydrochlorid die sogenannte Verunreinigung A. Wie eingangs gleichermaßen geschildert, sind die typischen Abbauprodukte des Wirkstoffs bzw. der Komponente (b), insbesondere von Pantothenol, das Aminopropanol bzw. dessen Ammoniumsalz und D-Pantolacton.

Im Allgemeinen kann es im Rahmen der vorliegenden Erfindung vorgesehen sein, dass die Zusammensetzung nach der Erfindung bei Temperaturen im Bereich von 20 °C bis 50 °C, bei einem Druck von 1.013,25 mbar und bei einer relativen Luftfeuchtigkeit im Bereich von 50 % bis 90 % mindestens 6 Monate, insbesondere mindestens 12 Monate, vorzugsweise mindestens 24 Monate, bevorzugt mindestens 36 Monate, besonders bevorzugt mindestens 39 Monate, ganz besonders bevorzugt mindestens 42 Monate stabil, insbesondere lagerstabil, ist.

Darüber hinaus verhält es sich erfindungsgemäß insbesondere derart, dass die Zusammensetzung nach der Erfindung ein Gehalt an Abbauprodukt(en) des Wirkstoffs (a) bzw. der Komponente (a), insbesondere Verunreinigung A, von höchstens 10 Gew.-%, insbesondere von höchstens 5 Gew.-%, vorzugsweise von höchstens 3 Gew.-%, bevorzugt von höchstens 2 Gew.-%, besonders bevorzugt von höchstens 1 Gew.-%, ganz besonders bevorzugt von höchstens 0,9 Gew.-%, bezogen auf den Wirkstoff (a) bzw. die Komponente (a), enthält, insbesondere auch nach Lagerung der Zusammensetzung bei Temperaturen im Bereich von 20 °C bis 50 °C, bei einem Druck von 1.013,25 mbar und bei einer relativen Luftfeuchtigkeit im Bereich von 50 % bis 90 % von mindestens 6 Monaten, insbesondere mindestens 12 Monaten, vorzugsweise mindestens 24 Monaten, bevorzugt mindestens 36 Monaten, besonders bevorzugt mindestens 39 Monaten, ganz besonders bevorzugt mindestens 42 Monaten.

Weiterhin kann es erfindungsgemäß vorgesehen sein, dass die Zusammensetzung nach der Erfindung einen Gehalt an Abbauprodukt(en) des Wirkstoffs (b) bzw. der Komponente (b), insbesondere Aminopropanol und/oder D-Pantolacton, von jeweils höchstens 10 Gew.-%, insbesondere von jeweils höchstens 5 Gew.-%, vorzugsweise von jeweils höchstens 3 Gew.-%, bevorzugt von jeweils höchstens 2 Gew.-%, besonders bevorzugt von jeweils höchstens 1 Gew.-%, ganz besonders bevorzugt von höchstens 0,9 Gew.-%, bezogen auf den Wirkstoff (b) bzw. die Komponente (b), enthält, insbesondere auch nach Lagerung der Zusammensetzung bei Temperaturen im Bereich von 20 °C bis 50 °C, bei einem Druck von 1.013,25 mbar und bei einer relativen Luftfeuchtigkeit im Bereich von 50 % bis 90 % von mindestens 6 Monaten, insbesondere mindestens 12 Monaten, vorzugsweise mindestens 24 Monaten, bevorzugt mindestens 36 Monaten, besonders bevorzugt mindestens 39 Monaten, ganz besonders bevorzugt mindestens 42 Monaten.

Insbesondere kann die Zusammensetzung eine Stabilität, insbesondere Lagerstabilität, unter beschleunigten Alterungsbedingungen gemäß ASTM F 1980 [ASTM F 1980: Standard Guide for Accelerated Aging of Sterile Barrier Systems for Medical Devices; 2007-04] bei einer Alterungstemperatur von 55 °C von mindestens 6 Monaten, insbesondere mindestens 12 Monaten, vorzugsweise mindestens 24 Monaten, bevorzugt mindestens 36 Monaten, besonders bevorzugt mindestens 39 Monaten, ganz besonders bevorzugt mindestens 42 Monaten, aufweisen. Insbesondere kann die Zusammensetzung in diesem Zusammenhang zum jeweiligen Lagerungszeitpunkt einen Gehalt an Abbauprodukten des Wirkstoffs (a) bzw. der Komponente (a) von höchstens 10 Gew.-%, insbesondere höchstens 5 Gew.-%, vorzugsweise höchstens 4 Gew.-%, bevorzugt höchstens 3 Gew.-%, besonders bevorzugt höchstens 2 Gew.-%, ganz besonders bevorzugt höchstens 1 Gew.-%, bezogen auf den Wirkstoff (a) bzw. die Komponente (a) bzw. das Edukt, aufweisen. Gleichermaßen kann die Zusammensetzung in diesem Zusammenhang zum jeweiligen Lagerungszeitpunkt einen Gesamtgehalt an Abbauprodukten der Komponente (b), insbesondere Aminopropanol und/oder D-Pantolacton, von höchstens 10 Gew.-%, insbesondere höchstens 5 Gew.-%, vorzugsweise höchstens 4 Gew.-%, bevorzugt höchstens 3 Gew.-%, besonders bevorzugt höchstens 2 Gew.-%, ganz besonders bevorzugt höchstens 1 Gew.-%, bezogen auf den Wirkstoff (b) bzw. die Komponente (b) bzw. das Edukt, aufweisen.

Insbesondere betrifft die vorliegende Erfindung auch eine wie zuvor geschilderte Zusammensetzung zur Verwendung bei der prophylaktischen bzw. kurativen topischen Behandlung von Rhinitis, insbesondere *Rhinitis acuta.*

Gleichermaßen betrifft die vorliegende Erfindung eine wie zuvor geschilderte Zusammensetzung zur prophylaktischen bzw. kurativen topischen Behandlung von Rhinitis, insbesondere *Rhinitis acuta.*

Was die erfindungsgemäße Zusammensetzung weiterhin anbelangt, so kann die zugrundeliegende Rhinitis insbesondere ausgewählt sein aus der Gruppe von *Rhinitis acuta, Rhinitis allergica, Rhinitis atrophicans, Rhinitis hyperplastica* oder *hypertrophicans, Rhinitis mutilans, Rhinitis nervosa* oder *vasomotorica, Rhinitis pseudomembranacea* und umweltbedingter Rhinitis, vorzugsweise *Rhinitis acuta.*

In diesem Zusammenhang betrifft die vorliegende Erfindung eine wie zuvor geschilderte Zusammensetzung zur Verwendung bei der prophylaktischen und/oder kurativen topischen Behandlung von Rhinitis, insbesondere *Rhinitis acuta, Rhinitis allergica, Rhinitis atrophicans, Rhinitis hyperplastica* oder *hypertrophicans, Rhinitis mutilans, Rhinitis nervosa* oder *vasomotorica, Rhinitis pseudomembranacea* oder umweltbedingter Rhinitis, vorzugsweise *Rhinitis acuta,* und/oder wobei die Rhinitis ausgewählt ist aus der Gruppe von *Rhinitis acuta, Rhinitis allergica, Rhinitis atrophicans, Rhinitis hyperplastica* oder *hypertrophicans, Rhinitis mutilans, Rhinitis nervosa* oder *vasomotorica* oder *Rhinitis pseudomembranacea* und umweltbedingter Rhinitis, vorzugsweise *Rhinitis acuta.*

Im Allgemeinen betrifft die vorliegende Erfindung gleichermaßen eine wie zuvor geschilderte Zusammensetzung zur prophylaktischen bzw. kurativen topischen Behandlung von Rhinitiden aller Art bzw. zur Verwendung bei der prophylaktischen und/oder kurativen topischen Behandlung von Rhinitiden aller Art.

Darüber hinaus betrifft die vorliegende Erfindung - gemäß einem **weiteren** Aspekt der vorliegenden Erfindung - eine Applikationsvorrichtung, welche eine wie zuvor beschriebene Zusammensetzung nach der vorliegenden Erfindung enthält, wobei die erfindungsgemäße Applikationsvorrichtung insbesondere für die topische, insbesondere nasale, vorzugsweise intranasale Applikation, geeignet ist und bevorzugt in Form eines Behältnisses mit Tropf- oder Sprüheinrichtung vorliegt.

Erfindungsgemäß ist es bevorzugt, wenn die erfindungsgemäße Applikationsvorrichtung eine Sprüheinrichtung zur gleichförmigen Ausbringung der Zusammensetzung nach der Erfindung in einer Menge pro Sprühstoß im Bereich von 25 µl bis 300 µl, insbesondere im Bereich von 50 µl bis 200 µl, vorzugsweise im Bereich von 75 µl bis 125 µl, aufweist.

Erfindungsgemäß ist es dabei insbesondere vorgesehen, dass die Applikationsvorrichtung nach der Erfindung ein Behältnis bzw. einen Vorratsbehälter mit einem Volumen im Bereich von 5 ml bis 100 ml, insbesondere 10 ml bis 50 ml, aufweist.

Dieses Behältnis bzw. dieser Vorratsbehälter dient insbesondere zur Aufnahme der erfindungsgemäßen Zusammensetzung.

Für weitergehende Einzelheiten zu der erfindungsgemäßen Applikationsvorrichtung kann auf die Ausführungen zu den weiteren Aspekten der vorliegenden Erfindung verwiesen werden, welche für die erfindungsgemäße Applikationsvorrichtung in entsprechender Weise gelten.

Weiterer Gegenstand der vorliegenden Erfindung - gemäß einem **weiteren** Aspekt der vorliegenden Erfindung - ist zudem die erfindungsgemäße Verpackungseinheit, welche mindestens eine Applikationsvorrichtung, wie zuvor definiert, enthält. In diesem Zusammenhang liegt die Applikationsvorrichtung in einer vor Kontamination schützenden Umverpackung eingebracht vor, insbesondere wobei die Applikationsvorrichtung in einer vor Kontaminationen schützenden Umverpackung eingebracht vorliegt.

Für weitergehende Einzelheiten zu der erfindungsgemäßen Verpackungseinheit kann auf die Ausführungen zu den weiteren Aspekten der vorliegenden Erfindung verwiesen werden, welche für die erfindungsgemäße Verpackungseinheit in entsprechender Weise gelten.

Darüber hinaus ist vorliegend die Verwendung der zuvor beschriebenen Zusammensetzung nach der Erfindung zur prophylaktischen bzw. kurativen topischen Behandlung von Rhinitis bzw. die Verwendung der zuvor beschriebenen Zusammensetzung nach der Erfindung zur Herstellung eines Arzneimittels zur prophylaktischen bzw. kurativen topischen Behandlung von Rhinitis beschrieben.

Was die vorliegend beschriebene Verwendung anbelangt, so bezieht sich diese insbesondere auf die Behandlung von *Rhinitis acuta, Rhinitis allergica, Rhinitis atrophicans, Rhinitis hyperplastica* oder *hypertrophicans, Rhinitis mutilans, Rhinitis nervosa* oder *vasomotorica* oder *Rhinitis pseudomembranacea* oder umweltbedingter Rhinitis, vorzugsweise *Rhinitis acuta.* In diesem Zusammenhang kann im Rahmen der vorliegend beschriebenen Verwendung somit die Rhinitis ausgewählt werden aus der Gruppe von *Rhinitis acuta, Rhinitis allergica, Rhinitis atrophicans, Rhinitis hyperplastica* oder *hypertrophicans, Rhinitis mutilans, Rhinitis nervosa* oder *vasomotorica* oder *Rhinitis pseudomembranacea* und umweltbedingter Rhinitis, vorzugsweise *Rhinitis acuta.*

Im Allgemeinen betrifft die vorliegend beschriebene Verwendung somit auch die vorliegenden Verwendungen zur Behandlung von Rhinitiden aller Art.

Im Rahmen der vorliegend beschriebenen Verwendungen kann die pharmazeutische Zusammensetzung nach der Erfindung, wie zuvor definiert, insbesondere nasal, vorzugsweise intranasal, appliziert werden.

Für weitergehende Einzelheiten zu den vorliegend beschriebenen Verwendungen kann auch auf die weiteren Ausführungen zu den übrigen Erfindungsaspekten verwiesen werden, welche in Bezug auf die vorliegend beschriebenen Verwendungen entsprechend gelten.

Weiterer Gegenstand der vorliegenden Erfindung - gemäß einem **weiteren** Aspekt der vorliegenden Erfindung - ist zudem auch Hyaluronsäure bzw. physiologisch verträgliches Hyaluronsäure-Salz (Hyaluronat), insbesondere in Form eines Alkalisalzes der Hyaluronsäure, besonders bevorzugt in Form eines Natriumsalzes der Hyaluronsäure, zur Verwendung bei der topischen, insbesondere nasalen, bevorzugt intranasalen Behandlung von Rhinitis,
wobei die Hyaluronsäure und/oder das physiologisch verträgliche Hyaluronsäure-Salz (Hyaluronat) in Kombination und/oder in gemeinsamer Zusammensetzung mit
(a) Xylometazolin, insbesondere in Form von dessen physiologisch verträglichen Salzen, besonders bevorzugt Xylometazolinhydrochlorid (Komponente (a)); und
(b) Pantothenol oder dessen physiologisch unbedenkliche Ester (Komponente (b));
   vorliegt und/oder eingesetzt wird und
   wobei die Hyaluronsäure und/oder das physiologisch verträgliche Hyaluronsäure-Salz ein zahlenmittleres Molekulargewicht (Molmasse) Mₙ im Bereich von 0,5 MDa bis 5 MDa und/oder ein gewichtsmittleres Molekulargewicht (Molmasse) M_{w} im Bereich von 0,6 MDa bis 6 MDa aufweist.

Wie zuvor angeführt, wird im Hinblick auf die Hyaluronsäure bzw. das diesbezügliche Hyaluronsäure-Salz, wie zuvor definiert, eine hohe Wirksamkeit bzw. gesteigerte Wirkeffizienz bei gleichzeitig guter Verträglichkeit bei der Behandlung der zuvor angeführten Rhinitiden gewährleistet.

In diesem Zusammenhang betrifft die vorliegende Erfindung gemäß diesem Aspekt der vorliegenden Erfindung insbesondere auch Hyaluronsäure bzw. physiologisch verträgliches Hyaluronsäure-Salz (Hyaluronat), insbesondere in Form eines Alkalisalzes der Hyaluronsäure, besonders bevorzugt in Form eines Natriumsalzes der Hyaluronsäure, zur Verwendung bei der topischen, insbesondere nasalen, bevorzugt intranasalen Behandlung von Rhinitis,
wobei die Hyaluronsäure und/oder das physiologisch verträgliche Hyaluronsäure-Salz (Hyaluronat) in Kombination und/oder in gemeinsamer Zusammensetzung mit
(a) Xylometazolin, insbesondere in Form von dessen physiologisch verträglichen Salzen, besonders bevorzugt Xylometazolinhydrochlorid (Komponente (a));
(b) Pantothenol oder dessen physiologisch unbedenkliche Ester, insbesondere Pantothenol, besonders bevorzugt Dexpanthenol (Komponente (b));
   vorliegt und/oder eingesetzt wird und
   wobei die Hyaluronsäure bzw. das physiologisch verträgliche Hyaluronsäure-Salz (Komponente (c)) ein zahlenmittleres Molekulargewicht (Molmasse) Mₙ im Bereich von 0,5 MDa bis 5 MDa und/oder ein gewichtsmittleres Molekulargewicht (Molmasse) M_{w} im Bereich von 0,6 MDa bis 6 MDa aufweist.

Für weitergehende Einzelheiten zu der erfindungsgemäßen Hyaluronsäure bzw. zu dem diesbezüglichen Hyaluronsäure-Salz (Hyaluronat) und der zugrundeliegenden Zusammensetzung kann auch auf die weitergehenden Ausführungen zu den weiteren erfindungsgemäßen Aspekten verwiesen werden, welche im Hinblick auf die Hyaluronsäure bzw. das erfindungsgemäße physiologisch verträgliche Hyaluronsäure-Salz (Hyaluronat) gemäß dem vorliegenden Aspekt entsprechend gelten.

Weiterer Gegenstand der vorliegenden Erfindung ist zudem - gemäß einem **weiteren** Aspekt der vorliegenden Erfindung - auch das erfindungsgemäße Verfahren zur Stabilisierung (Erhöhung der Stabilität), insbesondere zur Erhöhung der Lagerstabilität, und/oder zur Verbesserung der Verträglichkeit und/oder zur Erhöhung der Wirksamkeit, insbesondere der Wirkdauer, einer Zusammensetzung, insbesondere pharmazeutischen Zusammensetzung, vorzugsweise wie zuvor definiert, insbesondere wobei die Zusammensetzung als wässrige Zusammensetzung vorliegt, wobei die Zusammensetzung in Kombination, vorzugsweise in gemeinsamer wässriger Lösung, und jeweils in wirksamen, insbesondere pharmazeutisch wirksamen Mengen
(a) Xylometazolin, insbesondere in Form von dessen physiologisch verträglichen Salzen, besonders bevorzugt Xylometazolinhydrochlorid (Komponente (a));
(b) Pantothenol oder dessen physiologisch unbedenkliche Ester (Komponente (b));
   enthält,
   durch Zugabe von (c) Hyaluronsäure und/oder eines physiologisch verträglichen Hyaluronsäure-Salzes (Hyaluronat), insbesondere in Form eines Alkalisalzes der Hyaluronsäure, besonders bevorzugt in Form eines Natriumsalzes der Hyaluronsäure (Komponente (c)), zu der Zusammensetzung,
   wobei die Hyaluronsäure und/oder das physiologisch verträgliche Hyaluronsäure-Salz (Komponente (c)) ein zahlenmittleres Molekulargewicht (Molmasse) Mₙ im Bereich von 0,5 MDa bis 5 MDa und/oder ein gewichtsmittleres Molekulargewicht (Molmasse) M_{w} im Bereich von 0,6 MDa bis 6 MDa aufweist.

Denn die Anmelderin hat gleichermaßen in völlig überraschender Weise gefunden, dass, wie zuvor angeführt, durch den zweckgerichteten Einsatz einer speziellen Hyaluronsäure bzw. eines speziellen Hyaluronsäure-Salzes mit definiertem Molekulargewicht eine nachhaltige Stabilisierung der zugrundeliegenden Zusammensetzung nach der Erfindung gewährleistet wird, so dass deren Lagerstabilität erhöht wird, einhergehend mit einer weiterführenden Verbesserung der Handhabung sowie der Anwendungssicherheit. Infolgedessen vereint die erfindungsgemäße Zusammensetzung die Vorteile einer hohen Wirksamkeit bzw. Wirkeffizienz einerseits und einer hohen Stabilität, insbesondere Lagerstabilität, andererseits in ein und derselben Zusammensetzung.

Gemäß dem vorliegenden Aspekt betrifft die vorliegende Erfindung gleichermaßen auch ein Verfahren zur Stabilisierung (Erhöhung der Stabilität), insbesondere zur Erhöhung der Lagerstabilität, und/oder zur Verbesserung der Verträglichkeit und/oder zur Erhöhung der Wirksamkeit, insbesondere der Wirkdauer, einer Zusammensetzung, insbesondere pharmazeutischen Zusammensetzung, vorzugsweise wie zuvor definiert, insbesondere wobei die Zusammensetzung als wässrige Zusammensetzung vorliegt,
wobei die Zusammensetzung in Kombination, vorzugsweise in gemeinsamer wässriger Lösung, und jeweils in wirksamen, insbesondere pharmazeutisch wirksamen Mengen
(a) Xylometazolin, insbesondere in Form von dessen physiologisch verträglichen Salzen, besonders bevorzugt Xylometazolinhydrochlorid (Komponente (a));
(b) Pantothenol oder dessen physiologisch unbedenkliche Ester, insbesondere Pantothenol, besonders bevorzugt Dexpanthenol (Komponente (b));
   enthält,
   durch Zugabe von (c) Hyaluronsäure und/oder eines physiologisch verträglichen Hyaluronsäure-Salzes (Hyaluronat), insbesondere in Form eines Alkalisalzes der Hyaluronsäure, besonders bevorzugt in Form eines Natriumsalzes der Hyaluronsäure (Komponente (c)), zu der Zusammensetzung,
   wobei die Hyaluronsäure und/oder das physiologisch verträgliche Hyaluronsäure-Salz (Komponente (c)) ein zahlenmittleres Molekulargewicht (Molmasse) Mₙ im Bereich von 0,5 MDa bis 5 MDa und/oder ein gewichtsmittleres Molekulargewicht (Molmasse) M_{w} im Bereich von 0,6 MDa bis 6 MDa aufweist.

In diesem Zusammenhang betrifft die vorliegende Erfindung auch ein Verfahren zur Stabilisierung (Erhöhung der Stabilität), insbesondere zur Erhöhung der Lagerstabilität, und/oder zur Verbesserung der Verträglichkeit und/oder zur Erhöhung der Wirksamkeit, insbesondere der Wirkdauer, einer Komponente (a) (Wirkstoff (a)) und/oder einer Komponente (b) (Wirkstoff (b)) in einer Zusammensetzung, insbesondere pharmazeutischen Zusammensetzung, vorzugsweise wie zuvor definiert, insbesondere wobei die Zusammensetzung als wässrige Zusammensetzung vorliegt,
wobei die Komponente (a) Xylometazolin, insbesondere in Form von dessen physiologisch verträglichen Salzen, besonders bevorzugt Xylometazolinhydrochlorid, ist und
wobei die Komponente (b) Pantothenol oder dessen physiologisch unbedenkliche Ester ist,
durch Zugabe von (c) Hyaluronsäure und/oder eines physiologisch verträglichen Hyaluronsäure-Salzes (Hyaluronat), insbesondere in Form eines Alkalisalzes der Hyaluronsäure, besonders bevorzugt in Form eines Natriumsalzes der Hyaluronsäure (Komponente (c)), zu der Zusammensetzung,
wobei die Hyaluronsäure und/oder das physiologisch verträgliche Hyaluronsäure-Salz (Komponente (c)) ein zahlenmittleres Molekulargewicht (Molmasse) Mₙ im Bereich von 0,5 MDa bis 5 MDa und/oder ein gewichtsmittleres Molekulargewicht (Molmasse) M_{w} im Bereich von 0,6 MDa bis 6 MDa aufweist.

In diesem Zusammenhang betrifft die vorliegende Erfindung auch ein solches Verfahren zur Stabilisierung (Erhöhung der Stabilität), insbesondere zur Erhöhung der Lagerstabilität, und/oder zur Verbesserung der Verträglichkeit und/oder zur Erhöhung der Wirksamkeit, insbesondere der Wirkdauer, einer Komponente (a) (Wirkstoff (a)) und/oder einer Komponente (b) (Wirkstoff (b)) in einer Zusammensetzung, insbesondere pharmazeutischen Zusammensetzung, vorzugsweise wie zuvor definiert, insbesondere wobei die Zusammensetzung als wässrige Zusammensetzung vorliegt und/oder insbesondere wobei die Komponente (a) und die Komponente (b) in Kombination und/oder in gemeinsamer wässriger Lösung in der Zusammensetzung vorliegen, vorzugsweise jeweils in wirksamen, insbesondere pharmazeutisch wirksamen Mengen,
wobei die Komponente (a) Xylometazolin, insbesondere in Form von dessen physiologisch verträglichen Salzen, besonders bevorzugt Xylometazolinhydrochlorid, ist und
wobei die Komponente (b) Pantothenol oder dessen physiologisch unbedenkliche Ester, insbesondere Pantothenol, besonders bevorzugt Dexpanthenol, ist,
durch Zugabe von (c) Hyaluronsäure und/oder eines physiologisch verträglichen Hyaluronsäure-Salzes (Hyaluronat), insbesondere in Form eines Alkalisalzes der Hyaluronsäure, besonders bevorzugt in Form eines Natriumsalzes der Hyaluronsäure (Komponente (c)), zu der Zusammensetzung,
wobei die Hyaluronsäure und/oder das physiologisch verträgliche Hyaluronsäure-Salz (Komponente (c)) ein zahlenmittleres Molekulargewicht (Molmasse) Mₙ im Bereich von 0,5 MDa bis 5 MDa und/oder ein gewichtsmittleres Molekulargewicht (Molmasse) M_{w} im Bereich von 0,6 MDa bis 6 MDa aufweist.

Für weitergehende Einzelheiten zu den erfindungsgemäßen Verfahren kann auch auf die weiteren Ausführungen zu den übrigen Aspekten der vorliegenden Erfindung verwiesen werden, welche im Hinblick auf die erfindungsgemäßen Verfahren gemäß dem vorliegenden Aspekt entsprechend gelten.

Darüber hinaus ist weiterer Gegenstand der vorliegenden Erfindung - gemäß einem **weiteren** Aspekt der vorliegenden Erfindung - zudem auch die Verwendung von Hyaluronsäure und/oder eines physiologisch verträglichen Hyaluronsäure-Salzes (Hyaluronat), insbesondere in Form eines Alkalisalzes der Hyaluronsäure, besonders bevorzugt in Form eines Natriumsalzes der Hyaluronsäure,
zur Stabilisierung (Erhöhung der Stabilität), insbesondere zur Erhöhung der Lagerstabilität, und/oder zur Verbesserung der Verträglichkeit und/oder zur Erhöhung der Wirksamkeit, insbesondere der Wirkdauer, einer Zusammensetzung, insbesondere pharmazeutischen Zusammensetzung, vorzugsweise wie zuvor definiert, insbesondere wobei die Zusammensetzung als wässrige Zusammensetzung vorliegt,
wobei die Zusammensetzung in Kombination, vorzugsweise in gemeinsamer wässriger Lösung, und jeweils in wirksamen, insbesondere pharmazeutisch wirksamen Mengen
(a) Xylometazolin, insbesondere in Form von dessen physiologisch verträglichen Salzen, besonders bevorzugt Xylometazolinhydrochlorid (Komponente (a));
(b) Pantothenol oder dessen physiologisch unbedenkliche Ester (Komponente (b));
   enthält,
   wobei die Hyaluronsäure und/oder das physiologisch verträgliche Hyaluronsäure-Salz (Hyaluronat) zu der Zusammensetzung zugegeben wird,
   wobei die Hyaluronsäure und/oder das physiologisch verträgliche Hyaluronsäure-Salz (Komponente (c)) ein zahlenmittleres Molekulargewicht (Molmasse) Mₙ im Bereich von 0,5 MDa bis 5 MDa und/oder ein gewichtsmittleres Molekulargewicht (Molmasse) M_{w} im Bereich von 0,6 MDa bis 6 MDa aufweist.

In diesem Zusammenhang betrifft die vorliegende Erfindung auch die Verwendung von Hyaluronsäure und/oder eines physiologisch verträglichen Hyaluronsäure-Salzes (Hyaluronat), insbesondere in Form eines Alkalisalzes der Hyaluronsäure, besonders bevorzugt in Form eines Natriumsalzes der Hyaluronsäure,
zur Stabilisierung (Erhöhung der Stabilität), insbesondere zur Erhöhung der Lagerstabilität, und/oder zur Verbesserung der Verträglichkeit und/oder zur Erhöhung der Wirksamkeit, insbesondere der Wirkdauer, einer Zusammensetzung, insbesondere pharmazeutischen Zusammensetzung, vorzugsweise wie zuvor definiert, insbesondere wobei die Zusammensetzung als wässrige Zusammensetzung vorliegt,
wobei die Zusammensetzung in Kombination, vorzugsweise in gemeinsamer wässriger Lösung, und jeweils in wirksamen, insbesondere pharmazeutisch wirksamen Mengen
(a) Xylometazolin, insbesondere in Form von dessen physiologisch verträglichen Salzen, besonders bevorzugt Xylometazolinhydrochlorid (Komponente (a));
(b) Pantothenol oder dessen physiologisch unbedenkliche Ester, insbesondere Pantothenol, besonders bevorzugt Dexpanthenol (Komponente (b));
   enthält,
   wobei die Hyaluronsäure und/oder das physiologisch verträgliche Hyaluronsäure-Salz (Hyaluronat) zu der Zusammensetzung zugegeben wird,
   wobei die Hyaluronsäure und/oder das physiologisch verträgliche Hyaluronsäure-Salz (Komponente (c)) ein zahlenmittleres Molekulargewicht (Molmasse) Mₙ im Bereich von 0,5 MDa bis 5 MDa und/oder ein gewichtsmittleres Molekulargewicht (Molmasse) M_{w} im Bereich von 0,6 MDa bis 6 MDa aufweist.

Für weitergehende Einzelheiten zu den erfindungsgemäßen Verwendungen kann auf die weiterführenden Ausführungen zu den weiteren Erfindungsaspekten verwiesen werden, welche im Hinblick auf die vorliegenden Verwendungen nach der Erfindung entsprechend gelten.

Im Rahmen der vorliegenden Erfindung wird somit insgesamt unter Zugrundelegung der erfindungsgemäßen Zusammensetzung mit dem gezielten Einsatz einer Hyaluronsäure bzw. eines Hyaluronsäure-Salzes mit hohem Molekulargewicht ein leistungsfähiges Gesamtkonzept zur Behandlung von Rhinitis, bevorzugt *Rhinitis acuta,* bereitgestellt, wobei die erfindungsgemäße Zusammensetzung neben einer hohen Wirksamkeit und Verträglichkeit auch eine signifikant erhöhte Stabilität, insbesondere Lagerungsstabilität bzw. Lagerstabilität, mit diesbezüglich hervorragender Handhabbarkeit verfügt, wobei diesbezüglich sogar auf den Einsatz von Konservierungsmitteln bzw. -stoffen bzw. Desinfektionsmitteln bzw. -stoffen verzichtet werden kann.

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.

Die folgenden Ausführungsbeispiele dienen lediglich der Veranschaulichung der vorliegenden Erfindung, ohne die vorliegende Erfindung jedoch hierauf zu beschränken.

### AUSFÜHRUNGSBEISPIELE

### 1. Herstellungsbeispiele:

### Allgemeine Herstellvorschrift

Zur Herstellung von jeweils 1.000 g einer klaren wässrigen Lösung der erfindungsgemäßen Zusammensetzung wird in dem Fachmann an sich bekannter Weise vorgegangen:
Zunächst wird bei Raumtemperatur und Umgebungsdruck in einem entsprechenden Glasgefäß mit Rühreinrichtung eine definierte Menge an gereinigtem Wasser (z. B. 300 ml bis 600 ml) vorgelegt und nachfolgend mit einer Lösung des gewählten Puffersystems auf einen vorgegeben pH-Wert im Bereich zwischen 5,0 und 6,0 (z. B. pH-Wert von ca. 5,5) unter anschließender Kontrolle des erreichten pH-Werts eingestellt. Anschließend wird hierzu die nachfolgend in den Rezepturbeispielen spezifizierte Dexpanthenolmenge hinzugegeben, und das Ganze wird dann durch gründliches Rühren klar gelöst. Nachfolgend wird die gewünschte Xylometazolinmenge zugesetzt. Der Lösung wird dann die Hyaluronsäure, bevorzugt in Form einer wässrigen Lösung des Hyaluronsäuresalzes (Natriumhyaluronat), zugesetzt und gleichfalls unter Rühren gelöst und mit weiterem Wasser zum Endgewicht von 1.000 g aufgefüllt und homogen gerührt. Gegebenenfalls wird die Lösung über neutrale Cellulosefilter filtriert. Abschließend wird der pH-Wert noch einmal kontrolliert. Auch die übrigen entsprechenden Spezifikationen der Lösung werden auf die eingestellten bzw. vorgewählten Wertebereiche hin überprüft (z. B. Osmolalität: 350 bis 495 mosmol/kg; relative Dichte bei 20 °C: 1,005 bis 1,105; mikrobiologische Reinheit und Sterilität; Ausschluss von Verunreinigungen, insbesondere Abbauprodukten der Inhalts- und Wirkstoffe).

Ein Teil der erhaltenen Lösung wird schließlich in Enghalsflaschen aus Braunglas zu 10 ml oder 20 ml abgefüllt, welche wahlweise mit einer Tropfpipette oder einer Sprühdosierpumpe ausgerüstet werden können; zur bestimmungsgemäßen Anwendung können mehrmals täglich ein bis drei Tropfen bzw. ein bis zwei Sprühstöße in jedes Nasenloch gegeben und aufgezogen werden. Ein anderer Teil der erhaltenen Lösung wird für die nachfolgend beschriebenen Stabilitätsuntersuchungen verwendet.

Nach dieser allgemeinen Herstellvorschrift werden die nachfolgend spezifizierten Zusammensetzungen bzw. Rezepturen mit den diesbezüglichen Mengenangaben (Einwaage bei Herstellung der Zusammensetzung) hergestellt:

### Zusammensetzung A1 (Angabe pro 10 g Zusammensetzung)

| **Inhaltsstoff** | **Menge / mg** | **Qualität** |
|---|---|---|
| Xylometazolinhydrochlorid | 10,00 | Ph. Eur. |
| Dexpanthenol | 500,00 | Ph. Eur. |
| Natriumhyaluronat | 2,00 | Ph. Eur. |
| Mₙ = 1,4 MDa; M_{w} = 1,6 MDa | | |
| Puffersystem: | | Ph. Eur. |
| Kaliumdihydrogenphosphat / | 85,30 | |
| Natriummonohydrogenphosphat (Dodekahydrat) | 2,70 | |
| Gereinigtes Wasser | 9.400,00 | Ph. Eur. |

Der pH-Wert der Zusammensetzung A1 beträgt zudem etwa 5,5. Weiterhin weist die vorliegende Zusammensetzung eine dynamische Viskosität von etwa 2,0 mPas auf, und die relative Dichte beträgt etwa 1,015 g/cm³ (20 °C und Atmosphärendruck). Die Osmolalität der vorliegenden Zusammensetzung beträgt zudem etwa 410 mosmol/kg.

### Zusammensetzung A2 (Angabe pro 10 g Zusammensetzung)

In entsprechender Weise wird eine weitere Zusammensetzung A2 hergestellt, wobei jedoch gegenüber der Zusammensetzung A1 ein Natriumhyluronat mit demgegenüber höherem Molekulargewicht (Molmasse) eingesetzt wird:

| **Inhaltsstoff** | **Menge / mg** | **Qualität** |
|---|---|---|
| Xylometazolinhydrochlorid | 10,00 | Ph. Eur. |
| Dexpanthenol | 500,00 | Ph. Eur. |
| Natriumhyaluronat | 2,00 | Ph. Eur. |
| Mₙ = 2,2 MDa; M_{w} = 2,3 MDa | | |
| Puffersystem: | | Ph. Eur. |
| Kaliumdihydrogenphosphat / | 85,30 | |
| Natriummonohydrogenphosphat (Dodekahydrat) | 2,70 | |
| Gereinigtes Wasser | 9.400,00 | Ph. Eur. |

### Zusammensetzungen B1 bis B4 (Angabe pro 10 g Zusammensetzung):

Die weiteren Zusammensetzungen B1 bis B8 entsprechen der Zusammensetzung A1 bzw. A2, jedoch mit der Maßgabe, dass das Natriumhyluronat jeweils mit hierzu unterschiedlichen Molekulargewichten eingesetzt wird, wie nachfolgend angeführt:

### Zusammensetzung B1

| **Inhaltsstoff** | **Menge** / **mg** | **Qualität** |
|---|---|---|
| Xylometazolinhydrochlorid | 10,00 | Ph. Eur. |
| Dexpanthenol | 500,00 | Ph. Eur. |
| Natriumhyaluronat | 2,00 | Ph. Eur. |
| Mₙ = 0,55 MDa; M_{w} = 0,65 MDa | | |
| Puffersystem: | | Ph. Eur. |
| Kaliumdihydrogenphosphat / | 85,30 | |
| Natriummonohydrogenphosphat (Dodekahydrat) | 2,70 | |
| Gereinigtes Wasser | 9.400,00 | Ph. Eur. |

### Zusammensetzung B2

| **Inhaltsstoff** | **Menge / mg** | **Qualität** |
|---|---|---|
| Xylometazolinhydrochlorid | 10,00 | Ph. Eur. |
| Dexpanthenol | 500,00 | Ph. Eur. |
| Natriumhyaluronat | 2,00 | Ph. Eur. |
| Mₙ = 4,5 MDa; M_{w} = 4,9 MDa | | |
| Puffersystem: | | Ph. Eur. |
| Kaliumdihydrogenphosphat / | 85,30 | |
| Natriummonohydrogenphosphat (Dodekahydrat) | 2,70 | |
| Gereinigtes Wasser | 9.400,00 | Ph. Eur. |

### Zusammensetzung B3 (Vergleich)

| **Inhaltsstoff** | **Menge / mg** | **Qualität** |
|---|---|---|
| Xylometazolinhydrochlorid | 10,00 | Ph. Eur. |
| Dexpanthenol | 500,00 | Ph. Eur. |
| Natriumhyaluronat | 2,00 | Ph. Eur. |
| Mₙ = 0,35 MDa; M_{w} = 0,45 MDa | | |
| Puffersystem: | | Ph. Eur. |
| Kaliumdihydrogenphosphat / | 85,30 | |
| Natriummonohydrogenphosphat (Dodekahydrat) | 2,70 | |
| Gereinigtes Wasser | 9.400,00 | Ph. Eur. |

### Zusammensetzung B4 (Vergleich)

| **Inhaltsstoff** | **Menge** / **mg** | **Qualität** |
|---|---|---|
| Xylometazolinhydrochlorid | 10,00 | Ph. Eur. |
| Dexpanthenol | 500,00 | Ph. Eur. |
| Natriumhyaluronat | 2,00 | Ph. Eur. |
| Mₙ = 5,8 MDa; M_{w} = 6,5 MDa | | |
| Puffersystem: | | Ph. Eur. |
| Kaliumdihydrogenphosphat / | 85,30 | |
| Natriummonohydrogenphosphat (Dodekahydrat) | 2,70 | |
| Gereinigtes Wasser | 9.400,00 | Ph. Eur. |

### Zusammensetzungen C1 bis C4

Es werden weitere Zusammensetzungen entsprechend der Zusammensetzung A1 hergestellt, jedoch mit der Maßgabe, dass unterschiedliche Mengen an Natriumhyaluronat eingesetzt werden bzw. dass kein Natriumhyaluronat eingesetzt wird:

### Zusammensetzung C1 (Vergleich)

| **Inhaltsstoff** | **Menge / mg** | **Qualität** |
|---|---|---|
| Xylometazolinhydrochlorid | 10,00 | Ph. Eur. |
| Dexpanthenol | 500,00 | Ph. Eur. |
| Puffersystem: | | Ph. Eur. |
| Kaliumdihydrogenphosphat / | 85,30 | |
| Natriummonohydrogenphosphat (Dodekahydrat) | 2,70 | |
| Gereinigtes Wasser | 9.402,00 | Ph. Eur. |

### Zusammensetzung C2

| **Inhaltsstoff** | **Menge / mg** | **Qualität** |
|---|---|---|
| Xylometazolinhydrochlorid | 10,00 | Ph. Eur. |
| Dexpanthenol | 500,00 | Ph. Eur. |
| Natriumhyaluronat | 1,00 | Ph. Eur. |
| Mₙ = 1,4 MDa; M_{w} = 1,6 MDa | | |
| Puffersystem: | | Ph. Eur. |
| Kaliumdihydrogenphosphat / | 85,30 | |
| Natriummonohydrogenphosphat (Dodekahydrat) | 2,70 | |
| Gereinigtes Wasser | 9.401,00 | Ph. Eur. |

### Zusammensetzung C3

| **Inhaltsstoff** | **Menge / mg** | **Qualität** |
|---|---|---|
| Xylometazolinhydrochlorid | 10,00 | Ph. Eur. |
| Dexpanthenol | 500,00 | Ph. Eur. |
| Natriumhyaluronat | 10,00 | Ph. Eur. |
| Mₙ = 1,4 MDa; M_{w} = 1,6 MDa | | |
| Puffersystem: | | Ph. Eur. |
| Kaliumdihydrogenphosphat / | 85,30 | |
| Natriummonohydrogenphosphat (Dodekahydrat) | 2,70 | |
| Gereinigtes Wasser | 9.392,00 | Ph. Eur. |

### Zusammensetzung C4

| **Inhaltsstoff** | **Menge / mg** | **Qualität** |
|---|---|---|
| Xylometazolinhydrochlorid | 10,00 | Ph. Eur. |
| Dexpanthenol | 500,00 | Ph. Eur. |
| Natriumhyaluronat | 100,00 | Ph. Eur. |
| Mₙ = 1,4 MDa; M_{w} = 1,6 MDa | | |
| Puffersystem: | | Ph. Eur. |
| Kaliumdihydrogenphosphat / | 85,30 | |
| Natriummonohydrogenphosphat (Dodekahydrat) | 2,70 | |
| Gereinigtes Wasser | 9.302,00 | Ph. Eur. |

### Zusammensetzungen D1 bis D4

Es werden weitere Zusammensetzungen entsprechend der Zusammensetzung A1 hergestellt, jedoch mit der Maßgabe, dass der pH-Wert wie folgt eingestellt wird:

| **Zusammensetzung** | **pH-Wert** |
|---|---|
| Zusammensetzung D1 | 4,0 |
| Zusammensetzung D2 | 4,6 |
| Zusammensetzung D3 | 5,0 |
| Zusammensetzung D4 | 6,0 |
| Zusammensetzung D5 | 6,3 |
| Zusammensetzung D6 | 7,0 |

### Zusammensetzungen E1 bis E4

Bei wiederum weiteren Zusammensetzungen, welche in Anlehnung an Zusammensetzung A1 hergestellt sind, wird zudem ein Konservierungs- bzw. Desinfektionsmittel eingesetzt, wie nachfolgend angeführt:

### Zusammensetzung E1

| **Inhaltsstoff** | **Menge / mg** | **Qualität** |
|---|---|---|
| Xylometazolinhydrochlorid | 10,00 | Ph. Eur. |
| Dexpanthenol | 500,00 | Ph. Eur. |
| Natriumhyaluronat Mₙ = 1,4 MDa; M_{w} = 1,6 MDa | 2,00 | Ph. Eur. |
| Puffersystem: | | Ph. Eur. |
| Kaliumdihydrogenphosphat / | 85,30 | |
| Natriummonohydrogenphosphat (Dodekahydrat) | 2,70 | |
| Konservierungs-/Desinfektionsmittel: | | Ph. Eur. |
| Benzalkoniumchlorid | 2,00 | |
| Gereinigtes Wasser | 9.398,00 | Ph. Eur. |

### Zusammensetzung E2

| **Inhaltsstoff** | **Menge / mg** | **Qualität** |
|---|---|---|
| Xylometazolinhydrochlorid | 10,00 | Ph. Eur. |
| Dexpanthenol | 500,00 | Ph. Eur. |
| Natriumhyaluronat | 2,00 | Ph. Eur. |
| Mₙ = 1,4 MDa; M_{w} = 1,6 MDa | | |
| Puffersystem: | | Ph. Eur. |
| Kaliumdihydrogenphosphat / | 85,30 | |
| Natriummonohydrogenphosphat (Dodekahydrat) | 2,70 | |
| Konservierungs-/Desinfektionsmittel: | | Ph. Eur. |
| Polyhexanid | 2,00 | |
| Gereinigtes Wasser | 9.398,00 | Ph. Eur. |

### Zusammensetzung E3

| **Inhaltsstoff** | **Menge / mg** | **Qualität** |
|---|---|---|
| Xylometazolinhydrochlorid | 10,00 | Ph. Eur. |
| Dexpanthenol | 500,00 | Ph. Eur. |
| Natriumhyaluronat | 2,00 | Ph. Eur. |
| Mₙ = 1,4 MDa; M_{w} = 1,6 MDa | | |
| Puffersystem: | | Ph. Eur. |
| Kaliumdihydrogenphosphat / | 85,30 | |
| Natriummonohydrogenphosphat (Dodekahydrat) | 2,70 | |
| Konservierungs-/Desinfektionsmittel: | | Ph. Eur. |
| Chlorhexidin | 2,00 | |
| Gereinigtes Wasser | 9.398,00 | Ph. Eur. |

### Zusammensetzung E4

| **Inhaltsstoff** | **Menge / mg** | **Qualität** |
|---|---|---|
| Xylometazolinhydrochlorid | 10,00 | Ph. Eur. |
| Dexpanthenol | 500,00 | Ph. Eur. |
| Natriumhyaluronat | 2,00 | Ph. Eur. |
| Mₙ = 1,4 MDa; M_{w} = 1,6 MDa | | |
| Puffersystem: | | Ph. Eur. |
| Kaliumdihydrogenphosphat / | 85,30 | |
| Natriummonohydrogenphosphat (Dodekahydrat) | 2,70 | |
| Konservierungs-/Desinfektionsmittel: | | Ph. Eur. |
| Sorbinsäure | 2,00 | |
| Gereinigtes Wasser | 9.398,00 | Ph. Eur. |

### Zusammensetzungen F1 bis F3

Es werden wiederum weitere Zusammensetzungen entsprechend der Zusammensetzung A1 hergestellt, jedoch mit der Maßgabe, dass diesbezüglich die nachfolgend angeführten chemischen Puffersysteme eingesetzt werden:

### Zusammensetzung F1

| **Inhaltsstoff** | **Menge / mg** | **Qualität** |
|---|---|---|
| Xylometazolinhydrochlorid | 10,00 | Ph. Eur. |
| Dexpanthenol | 500,00 | Ph. Eur. |
| Natriumhyaluronat | 2,00 | Ph. Eur. |
| Mₙ = 1,4 MDa; M_{w} = 1,6 MDa | | |
| Puffersystem: | | Ph. Eur. |
| Kohlensäure / Bicarbonat | 88,00 | |
| Gereinigtes Wasser | 9.400,00 | Ph. Eur. |

### Zusammensetzung F2

| **Inhaltsstoff** | **Menge / mg** | **Qualität** |
|---|---|---|
| Xylometazolinhydrochlorid | 10,00 | Ph. Eur. |
| Dexpanthenol | 500,00 | Ph. Eur. |
| Natriumhyaluronat | 2,00 | Ph. Eur. |
| Mₙ = 1,4 MDa; M_{w} = 1,6 MDa | | |
| Puffersystem: | | Ph. Eur. |
| Essigsäure / Acetat | 88,00 | |
| Gereinigtes Wasser | 9.400,00 | Ph. Eur. |

### Zusammensetzung F3

| **Inhaltsstoff** | **Menge / mg** | **Qualität** |
|---|---|---|
| Xylometazolinhydrochlorid | 10,00 | Ph. Eur. |
| Dexpanthenol | 500,00 | Ph. Eur. |
| Natriumhyaluronat | 2,00 | Ph. Eur. |
| Mₙ = 1,4 MDa; M_{w} = 1,6 MDa | | |
| Puffersystem: | | Ph. Eur. |
| Citronensäure / Citrat | 88,00 | |
| Gereinigtes Wasser | 9.400,00 | Ph. Eur. |

### 2. Stabilitätsuntersuchungen:

An den vorliegenden Zusammensetzungen bzw. Rezepturen werden entsprechende Stabilitätsuntersuchungen durchgeführt, wie nachfolgend im Detail angeführt.

Zu diesem Zweck werden jeweils unter definierten Lagerbedingungen Langzeitstabilitätsuntersuchungen an den betreffenden Zusammensetzungen durchgeführt, wobei zu Beginn der Untersuchungen und nach definierten Zeiträumen spezielle Prüfparameter an den Zusammensetzungen gemessen und mit der betreffenden Spezifikation verglichen wurden (Gehalt an Abbauprodukten bzw. Verunreinigungen), und zwar nach Lagerzeiten von 3 Monaten, 6 Monaten, 9 Monaten, 12 Monaten, 18 Monaten, 24 Monaten, 36 Monaten und 39 Monaten. Die Lagerung der Zusammensetzungen erfolgt bei einer definierten Temperatur von 30 °C ± 2 °C und bei einer relativen Luftfeuchte der Umgebung von 65 % ± 5 %.

Ein charakteristisches Maß für die Stabilität einer Zusammensetzung ist die Konstanz des Gehalts an Wirkstoffen (d. h. vorliegend Xylometazolinhydrochlorid sowie Dexpanthenol). In diesem Zusammenhang kann der Gehalt an Abbauprodukten der Wirkstoffe bestimmt werden, um die Stabilität der Zusammensetzung zu bewerten.

Wie eingangs geschildert, ist die sogenannte Verunreinigung A ein typisches Abbauprodukt des Xylometazolinhydrochlorids, während als Abbauprodukte des Dexpanthenols insbesondere auch Aminopropanol charakteristisch ist. In der nachfolgend angeführten Spezifikation des Gehaltes an Abbauprodukten bzw. Verunreinigungen beziehen sich die prozentualen Gehaltsangaben der Abbauprodukte bzw. Verunreinigungen jeweils als Gewichtsprozentangaben auf den ursprünglichen Wirkstoff, d. h. der Gehalt an Verunreinigung A als Gewichtsprozentangabe in Bezug auf Xylometazolinhydrochlorid und der Gehalt an Aminopropanol als Gewichtsprozentangabe in Bezug auf Dexpanthenol. Mit anderen Worten beziehen sich bei den Abbauprodukten bzw. Verunreinigungen die Gewichtsprozentangaben nicht auf die Zusammensetzung, sondern auf den Gehalt des betreffenden Wirkstoffs, aus dessen Abbau die betreffende Verunreinigung resultiert.

Die nachfolgenden Tabellen 1 bis 4 zeigen die ermittelten Ergebnisse der Stabilitätsuntersuchungen.

In Bezug auf die Verunreinigung A (in den Tabellen als "Ver. A" angeführt) bedeutet das Symbol "+", dass die zugrundeliegende Spezifikation, welche auf einen Gehalt an Verunreinigung A von höchstens 1 Gew.-%, bezogen auf den Wirkstoff (vorliegend Xylometazolinhydrochlorid), abstellt, zum Zeitpunkt der entsprechenden Lagerzeit erfüllt ist, während das Symbol "-" bedeutet, dass diese Spezifikation nicht erfüllt ist und somit ein Gehalt an Verunreinigung A von mehr als 1 Gew.-% vorliegt.

In Bezug auf das Abbauprodukt 3-Aminopropanol (in den Tabellen als "3-Amin." angeführt) bedeutet das Symbol "+", dass die zugrundeliegende Spezifikation, welche auf einen Gehalt an 3-Aminopropanol von höchstens 3 Gew.-%, bezogen auf den Wirkstoff (vorliegend Dexpanthenol), abstellt, zum Zeitpunkt der entsprechenden Lagerzeit erfüllt ist, während das Symbol "-" bedeutet, dass diese Spezifikation nicht erfüllt ist und somit ein Gehalt an 3-Aminopropanol von mehr als 3 Gew.-% vorliegt.

Zu den ermittelten Ergebnissen im Einzelnen:
a) Einfluss des Molekulargewichts der eingesetzten Hyaluronsäure

**(Tabelle 1):**

| | | **3 M** | **6M** | **9 M** | **12 M** | **18 M** | **24 M** | **36 M** | **39 M** |
|---|---|---|---|---|---|---|---|---|---|
| A1 | Ver. A | + | + | + | + | + | + | + | + |
| | 3-Amin. | + | + | + | + | + | + | + | + |
| A2 | Ver. A | + | + | + | + | + | + | + | + |
| | 3-Amin. | + | + | + | + | + | + | + | + |
| B1 | Ver. A | + | + | + | + | + | + | + | - |
| | 3-Amin. | + | + | + | + | + | + | + | - |
| B2 | Ver. A | + | + | + | + | + | + | + | + |
| | 3-Amin. | + | + | + | + | + | + | + | - |
| B3 | Ver. A | + | + | + | + | - | - | - | - |
| | 3-Amin. | + | + | + | + | + | - | - | - |
| B4 | Ver. A | + | + | + | + | + | - | - | - |
| | 3-Amin. | + | + | + | + | + | - | - | - |

| | | | | | | | | | |
|---|---|---|---|---|---|---|---|---|---|
| Tabelle 1 zeigt, dass dem Molekulargewicht der eingesetzten Hyaluronsäure eine nachhaltigen Wirkung auf die Stabilität der zugrundeliegenden Zusammensetzungen zukommt, nämlich dahingehend, dass für spezielle und in dem erfindungsgemäß angeführten Bereich liegende Molekulargewichte besonders hohe Stabilitäten (geringer Gehalt an Abbauprodukten bzw. Verunreinigungen über dem gesamten Lagerzeitraum) vorliegen, während für Werte außerhalb dieses Bereiches, also für Hyaluronsäuren mit relativ geringem Molekulargewicht bzw. mit relativ hohem Molekulargewicht, eine derartige Stabilisierung nicht erreicht werden kann. | | | | | | | | | |

b) Einfluss der Menge an eingesetzter Hyaluronsäure

**(Tabelle 2):**

| | | **3 M** | **6 M** | **9 M** | **12 M** | **18 M** | **24 M** | **36 M** | **39 M** |
|---|---|---|---|---|---|---|---|---|---|
| C1 | Ver. A | + | + | + | + | - | - | - | - |
| | 3-Amin. | + | + | + | - | - | - | - | - |
| C2 | Ver. A | + | + | + | + | + | + | + | - |
| | 3-Amin. | + | + | + | + | + | + | + | + |
| C3 | Ver. A | + | + | + | + | + | + | + | + |
| | 3-Amin. | + | + | + | + | + | + | + | + |
| C4 | Ver. A | + | + | + | + | + | + | + | + |
| | 3-Amin. | + | + | + | + | + | + | + | - |

| | | | | | | | | | |
|---|---|---|---|---|---|---|---|---|---|
| Tabelle 2 zeigt den Einfluss der eingesetzten Menge an Hyaluronsäure auf die Stabilitätseigenschaften der jeweiligen Zusammensetzungen, wonach bereits geringe Mengen an zugegebener Hyaluronsäure mit definiertem Molekulargewicht zu einer effektiven Stabilisierung bzw. Verbesserung der Lagerfähigkeit führen. Demgegenüber weist die Zusammensetzung ohne Hyaluronsäure gemäß der Zusammensetzung C1 keine derartig hohe Stabilität auf. | | | | | | | | | |

c) Einfluss des pH-Werts

**(Tabelle 3):**

| | | **3 M** | **6 M** | **9 M** | **12 M** | **18 M** | **24 M** | **36 M** | **39 M** |
|---|---|---|---|---|---|---|---|---|---|
| D1 | Ver. A | + | + | + | + | + | - | - | - |
| | 3-Amin. | + | + | + | + | + | - | - | - |
| D2 | Ver. A | + | + | + | + | + | + | - | - |
| | 3-Amin. | + | + | + | + | + | + | - | - |
| D3 | Ver. A | + | + | + | + | + | + | + | + |
| | 3-Amin. | + | + | + | + | + | + | + | + |
| D4 | Ver. A | + | + | + | + | + | + | + | + |
| | 3-Amin. | + | + | + | + | + | + | + | + |
| D5 | Ver. A | + | + | + | + | + | + | - | - |
| | 3-Amin. | + | + | + | + | + | + | - | - |
| D6 | Ver. A | + | + | + | + | + | - | - | - |
| | 3-Amin. | + | + | + | + | + | - | - | - |

| | | | | | | | | | |
|---|---|---|---|---|---|---|---|---|---|
| Tabelle 3 zeigt, dass dem den jeweiligen Zusammensetzungen zugrundeliegenden pH-Wert eine Bedeutung bei der Stabilisierung der Zusammensetzung zukommt. So weisen die Zusammensetzungen D3 und D4 (und diesbezüglich auch A1 bzw. A2) eine verbesserte Stabilität im Vergleich zu den Zusammensetzungen mit relativ niedrigem pH-Wert (Zusammensetzungen D1 und D2) bzw. mit relativ hohem pH-Wert (Zusammensetzungen D5 und D6) auf. Insbesondere fällt auf, dass die Stabilität der Zusammensetzungen D3 und D4 (und auch A1 bzw. A2) im Vergleich zu den weiteren Zusammensetzungen D1 und D2 bzw. D5 und D6 sozusagen sprunghaft verbessert ist, so dass diesbezüglich ein optimaler pH-Wert-Bereich mit weiterführender Erhöhung der Stabilität vorliegt. | | | | | | | | | |

d) Einfluss des Konservierungs- bzw. Desinfektionsmittels Weiterführende Untersuchungen der Anmelderin zeigen, dass die Verwendung eines Konservierungs- bzw. Desinfektionsmittels, wie in den Zusammensetzungen E1 bis E4 angeführt, mit einer Verschlechterung der Stabilitätseigenschaften einhergeht. Insbesondere kann bereits nach relativ kurzer Zeit eine Eintrübung der konservierungsmittel- bzw. desinfektionsmittelhaltigen Zusammensetzungen beobachtet werden, welche maßgeblich auf Ausfallprodukte zurückgeführt werden kann. Dabei weist die Zusammensetzung E4 mit der Verwendung von Sorbinsäure die diesbezüglich schlechtesten Eigenschaften auf (hohe und kurzfristig einsetzende Trübung). Ohne sich auf diese Theorie beschränken oder festlegen zu wollen, liegt im Fall der Verwendung von Konservierungs- bzw. Desinfektionsmittel gegebenenfalls eine der Stabilität abträgliche Wechselwirkung bzw. eine Inkompatibilität mit der speziellen Hyaluronsäure mit dem definierten hohen Molekulargewicht vor, was zu der aufgefundenen Verschlechterung der Stabilitätseigenschaften führt.
e) Einfluss des Puffersystems

**(Tabelle 4):**

| | | **3 M** | **6 M** | **9 M** | **12 M** | **18 M** | **24 M** | **36 M** | **39 M** |
|---|---|---|---|---|---|---|---|---|---|
| F1 | Ver. A | + | + | + | + | + | + | - | - |
| | 3-Amin. | + | + | + | + | + | + | - | - |
| F2 | Ver. A | + | + | + | + | + | + | - | - |
| | 3-Amin. | + | + | + | + | + | - | - | - |
| F3 | Ver. A | + | + | + | + | + | + | - | - |
| | 3-Amin. | + | + | + | + | + | - | - | - |

Im Vergleich zu der Zusammensetzung A1 bzw. A2 gehen die vorliegend eingesetzten Puffersysteme mit weniger hohen Stabilitäten der damit ausgerüsteten Zusammensetzungen einher. Insbesondere zeichnet sich demgegenüber das erfindungsgemäß in bevorzugter Weise eingesetzte Phosphatpuffersystem (vgl. insbesondere Zusammensetzungen A1 bzw. A2) gegenüber den anderen Puffersystemen dadurch aus, dass auf dieser Basis eine weiterführende Verbesserung der Langzeitstabilisierung erreicht werden kann. Ohne sich auf eine bestimmte Theorie beschränken oder festlegen zu wollen, lässt sich dieser Effekt des erfindungsgemäß bevorzugt eingesetzten Phosphatpuffersystems möglicherweise auf sekundäre Effekte, wie Komplexierungsreaktionen oder dergleichen, zurückführen, wie zuvor angeführt.

Die vorstehend referierten Ergebnisse der Stabilitätsuntersuchungen belegen den im Rahmen der vorliegenden Erfindung aufgefundenen Effekt einer weiterführenden Stabilisierung einer die Komponenten (a) und (b) enthaltenden Zusammensetzung durch den gezielten Einsatz einer speziellen Hyaluronsäure bzw. eines diesbezüglichen Hyaluronsäure-Salzes mit hohem Molekulargewicht gemäß Komponente (c), wie vorliegend definiert. Zudem zeigen die vorangehenden Untersuchungen auch, dass es zur Auffindung einer stabilen Zusammensetzung für die Behandlung von Rhinitis nach Art der vorliegenden Erfindung eines intensiven Forschungsaufwands seitens der Anmelderin bedurfte.

Die vorstehend referierten Stabilitätsuntersuchungen zeigen auch die Komplexität des von der Anmelderin aufgefundenen Lösungsansatzes. Hierzu zählen die Aspekte der gemeinsamen Anwesenheit der Wirkstoffe (a) und (b) einerseits sowie der Komponente (c) mit speziellem Molekulargewicht andererseits sowie weitere Co-Faktoren, wie insbesondere die Einhaltung bzw. Konstanthaltung des pH-Werts der Zusammensetzung im zuvor definierten Bereich, die Auswahl eines geeigneten Puffersystems, wobei eine zusätzliche Stabilitätsverbesserung erreicht werden kann, wenn keine Konservierungs- bzw. Desinfektionsmittel eingesetzt werden.

### 3. Versuche mit betroffenen Personen:

Je 15 betroffene Personen einer aus 30 Personen bestehenden Behandlungsgruppe im Alter von 24 bis 76 Jahren (19 männlich, 11 weiblich), welche an akuter Rhinitis litten, wurden mit der erfindungsgemäßen Zusammensetzung A1 oder aber A2 für die Dauer der Erkrankung (im Allgemeinen 3 bis 10 Tage) behandelt; die Zusammensetzungen bzw. Rezepturen wurden mehrmals täglich als Spray topisch appliziert. Weitere 10 Betroffene im Alter von 29 bis 72 Jahren (6 männlich, 4 weiblich) wurden demgegenüber mit der Zusammensetzung B3 (Hyaluronsäure mit geringem Molekulargewicht) behandelt, und wiederum weitere 10 Betroffene im Alter von 31 bis 73 Jahren (4 männlich, 6 weiblich) wurden mit der Zusammensetzung C1 (ohne Hyaluronsäure) mit vergleichbarem Verbreichungsschema behandelt. Der vasokonstriktorische Effekt von Xylometazolinhydrochlorid konnte, bezogen auf die Verbesserung der Symptomatik der Nasenschwellung und Behinderung der Nasenatmung, bei allen Betroffenen signifikant aufgezeigt werden, und zwar auch anhand von rhino(mano)metrischen Untersuchungen zur Bestimmung der Dekongestion bzw. Abschwellung. Zudem wurden insgesamt keine signifikanten Reizwirkungen der Nasenschleimhäute infolge des Xylometazolinhydrochlorids beobachtet. Die Anwendung der Zusammensetzungen A1 bzw. A2 ging darüber hinaus mit einer signifikant längeren Wirkdauer in Bezug auf die dekongestive Wirkung einher, und zwar mit einer Verlängerung von etwa 30 % im Vergleich zu der Hyaluronsäure-freien Zusammensetzung und etwa 15 % im Vergleich zu der Zusammensetzung mit der Hyaluronsäure mit niedrigem Molekulargewicht.

Die vorliegenden Behandlungsversuche zeigen somit insgesamt die hervorragende Wirksamkeit bei gleichzeitig guter Verträglichkeit der erfindungsgemäßen Zusammensetzung.

Mit den erfindungsgemäßen Zusammensetzungen konnten somit erfolgreich Behandlungsversuche mit betroffenen Personen mit *Rhinitis acuta* sowie weiterführende Stabilitätsuntersuchungen durchgeführt werden. Die erfindungsgemäßen Zusammensetzungen zeichnen sich durch eine hohe Wirksamkeit bei gleichzeitig guter Verträglichkeit und langer Wirkdauer sowie durch hervorragende Stabilitäten, insbesondere Lagerstabilitäten, aus.

Der erfindungsgemäße Lösungsansatz kommt im Ergebnis in überraschender Weise mit nur wenigen Inhaltsstoffen und vor allem ohne zusätzliche Konservierungsstoffe aus; dies ist im Hinblick auf die beabsichtigte pharmazeutische Wirkung, aber auch die Lagerstabilität von entscheidender Bedeutung.

## Patentansprüche

1. Zusammensetzung, insbesondere pharmazeutische Zusammensetzung, vorzugsweise für die topische, insbesondere nasale, bevorzugt intranasale Applikation, insbesondere für die Behandlung von Rhinitis,
wobei die Zusammensetzung in Kombination und jeweils in wirksamen, insbesondere pharmazeutisch wirksamen Mengen enthält:
(a) Xylometazolin, insbesondere in Form von dessen physiologisch verträglichen Salzen, besonders bevorzugt Xylometazolinhydrochlorid (Komponente (a));
(b) Pantothenol oder dessen physiologisch unbedenkliche Ester (Komponente (b));
(c) Hyaluronsäure und/oder ein physiologisch verträgliches Hyaluronsäure-Salz (Hyaluronat) (Komponente (c)),
wobei die Hyaluronsäure und/oder das physiologisch verträgliche Hyaluronsäure-Salz (Komponente (c)) ein zahlenmittleres Molekulargewicht (Molmasse) Mₙ im Bereich von 0,5 MDa bis 5 MDa und/oder ein gewichtsmittleres Molekulargewicht (Molmasse) M_{w} im Bereich von 0,6 MDa bis 6 MDa aufweist.

2. Zusammensetzung nach Anspruch 1,
wobei die Zusammensetzung die Komponente (c) und/oder die Hyaluronsäure und/oder das physiologisch verträgliche Hyaluronsäure-Salz in einer Menge im Bereich von 0,0001 Gew.-% bis 5 Gew.-%, insbesondere im Bereich von 0,0005 Gew.-% bis 3 Gew.-%, vorzugsweise im Bereich von 0,001 Gew.-% bis 1 Gew.-%, bevorzugt im Bereich von 0,005 Gew.-% bis 0,5 Gew.-%, besonders bevorzugt im Bereich von 0,01 Gew.-% bis 0,1 Gew.-%, bezogen auf die Zusammensetzung, enthält; und/oder
wobei die Komponente (c) und/oder die Hyaluronsäure und/oder das physiologisch verträgliche Hyaluronsäure-Salz ein zahlenmittleres Molekulargewicht (Molmasse) Mₙ im Bereich von 0,6 MDa bis 4,5 MDa, insbesondere im Bereich von 0,7 MDa bis 4 MDa, vorzugsweise im Bereich von 0,75 MDa bis 3,5 MDa, bevorzugt im Bereich von 0,8 MDa bis 3,25 MDa, besonders bevorzugt im Bereich von 0,9 MDa bis 3 MDa, aufweist; und/oder
wobei die Komponente (c) und/oder die Hyaluronsäure und/oder das physiologisch verträgliche Hyaluronsäure-Salz ein gewichtsmittleres Molekulargewicht (Molmasse) M_{w} im Bereich von 0,7 MDa bis 5,5 MDa, insbesondere im Bereich von 0,8 MDa bis 5 MDa, vorzugsweise im Bereich von 0,85 MDa bis 4,5 MDa, bevorzugt im Bereich von 0,9 MDa bis 4 MDa, besonders bevorzugt im Bereich von 0,95 MDa bis 3 MDa, aufweist; und/oder
wobei die Komponente (c) und/oder die Hyaluronsäure und/oder das physiologisch verträgliche Hyaluronsäure-Salz einen Polydispersitätsindex (PDI), berechnet als Quotient aus gewichtsmittlerem Molekulargewicht M_{w} und zahlenmittlerem Molekulargewicht Mₙ, im Bereich von 0,9 bis 10, insbesondere im Bereich von 0,95 bis 5, vorzugsweise im Bereich von 1 bis 3, bevorzugt im Bereich von 1,05 bis 2, besonders bevorzugt im Bereich von 1,1 bis 1,5, aufweist; und/oder
wobei die Hyaluronsäure und/oder das physiologisch verträgliche Hyaluronsäure-Salz (Komponente (c)) in Form von Natriumhyaluronat vorliegt.

3. Zusammensetzung nach Anspruch 1 oder 2,
wobei die Zusammensetzung die Komponente (a) und/oder das Xylometazolin in einer Menge im Bereich von 0,001 bis 2 Gew.-%, insbesondere im Bereich von 0,005 Gew.-% bis 1,5 Gew.-%, vorzugsweise im Bereich von 0,01 Gew.-% bis 1,2 Gew.-%, bevorzugt im Bereich von 0,02 Gew.-% bis 1,0 Gew.-%, besonders bevorzugt im Bereich von 0,03 Gew.-% bis 0,5 Gew.-%, ganz besonders bevorzugt im Bereich von 0,04 Gew.-% bis 0,2 Gew.-%, noch weiter bevorzugt im Bereich von 0,045 Gew.-% bis 0,175 Gew.-%, bezogen auf die Zusammensetzung, enthält; und/oder
wobei die Zusammensetzung die Komponente (b) und/oder Pantothenol oder dessen physiologisch unbedenkliche Ester in einer Menge im Bereich von 0,01 Gew.-% bis 10 Gew.-%, insbesondere im Bereich von 0,1 Gew.-% bis 9 Gew.-%, vorzugsweise im Bereich von 0,5 Gew.-% bis 8 Gew.-%, bevorzugt im Bereich von 1 Gew.-% bis 7 Gew.-%, besonders bevorzugt im Bereich von 2 Gew.-% bis 6 Gew.-%, ganz besonders bevorzugt im Bereich von 3 Gew.-% bis 6 Gew.-%, weiter bevorzugt im Bereich von 3,5 Gew.-% bis 5,5 Gew.-%, bezogen auf die Zusammensetzung, enthält.

4. Zusammensetzung nach einem der vorangehenden Ansprüche,
wobei die Zusammensetzung als wässrige Zusammensetzung vorliegt und/oder wobei die Zusammensetzung wässrig basiert ist und/oder wässrig formuliert vorliegt, insbesondere in Form einer wässrigen Lösung oder wässrigen Solubilisierung; und/oder
wobei die Zusammensetzung als wässriges System, insbesondere als wässriges Einphasensystem, vorzugsweise als wässrige Lösung oder wässrige Solubilisierung, vorliegt und/oder dass die Zusammensetzung einen Exzipienten oder Träger auf Wasserbasis und/oder in Form von Wasser aufweist und/oder dass die Zusammensetzung als klare, farblose wässrige Lösung vorliegt; und/oder wobei die Zusammensetzung frei von Konservierungsmitteln und/oder frei von Desinfektionsmitteln ist; und/oder
wobei der pH-Wert der Zusammensetzung wobei der pH-Wert der Zusammensetzung im Bereich von 4,9 bis 6,1, insbesondere im Bereich von 5,0 bis 6,0, vorzugsweise im Bereich von 5,1 bis 6,0, bevorzugt im Bereich von 5,2 bis 5,9, besonders bevorzugt im Bereich von 5,25 bis 5,85, eingestellt ist.

5. Zusammensetzung nach einem der vorangehenden Ansprüche,
wobei die Einstellung des pH-Werts der Zusammensetzung mittels mindestens eines chemischen Puffersystems, insbesondere Puffersalz(en) (Komponente (d)), erfolgt; und/oder
wobei die Zusammensetzung mindestens ein chemisches Puffersystem (Komponente (d)) aufweist.

6. Zusammensetzung nach Anspruch 5,
wobei die Zusammensetzung das chemische Puffersystem (Komponente (d)) in einer Menge im Bereich von 0,01 Gew.-% bis 5 Gew.-%, vorzugsweise im Bereich von 0,05 Gew.-% bis 4 Gew.-%, bevorzugt im Bereich von 0,1 Gew.-% bis 3 Gew.-%, besonders bevorzugt im Bereich von 0,5 Gew.-% bis 2 Gew.-%, bezogen auf die Zusammensetzung, enthält; und/oder
*wobei als chemisches Puffersystem (Komponente (d)) bevorzugt ein Dihydrogenphosphat*/*Monohydrogenposphat-Puffersystem ("H₂PO₄⁻*/*HPO₄²⁻*-*Puffer(system)" bzw. "Phosphatpuffer(system)"), insbesondere ein Alkalidihydrogenphosphat*/*Alkalimonohydrogenposphat-Puffersystem, eingesetzt wird; und*/*oder*
*wobei die Zusammensetzung als chemisches Puffersystem (Komponente (d)) bevorzugt ein Dihydrogenphosphat*/*Monohydrogenposphat-Puffersystem* (*"H₂PO₄⁻*/*HPO₄²⁻-Puffer-(system)" bzw. "Phosphatpuffer(system)"), insbesondere ein Alkalidihydrogenphosphat*/*Alkalimonohydrogenposphat-Puffersystem*, *enthält; und*/*oder*
wobei das Dihydrogenphosphat/Monohydrogenposphat-Puffersystem mit einem Dihydrogenphosphat/Monohydrogenposphat-Molverhältnis von größer 5:1, insbesondere im Bereich von 5 : 1 bis 110 : 1, besonders bevorzugt im Bereich von 6 : 1 bis 105:1, besonders bevorzugt im Bereich von 7:1 bis 100:1, ganz besonders bevorzugt im Bereich von 8 : 1 bis 90 : 1, eingesetzt wird; und/oder
wobei die Zusammensetzung das Dihydrogenphosphat/Monohydrogenposphat-Puffersystem mit einem Dihydrogenphosphat/Monohydrogenposphat-Molverhältnis von größer 5 : 1, insbesondere im Bereich von 5 : 1 bis 110 : 1, besonders bevorzugt im Bereich von 6 : 1 bis 105 : 1, besonders bevorzugt im Bereich von 7 : 1 bis 100 : 1, ganz besonders bevorzugt im Bereich von 8 : 1 bis 90 : 1, enthält.

7. Zusammensetzung nach einem der vorangehenden Ansprüche,
wobei die Zusammensetzung bei Temperaturen im Bereich von 20 °C bis 50 °C, bei einem Druck von 1.013,25 mbar und bei einer relativen Luftfeuchtigkeit im Bereich von 50 % bis 90 % mindestens 6 Monate, insbesondere mindestens 12 Monate, vorzugsweise mindestens 24 Monate, bevorzugt mindestens 36 Monate, besonders bevorzugt mindestens 39 Monate, ganz besonders bevorzugt mindestens 42 Monate stabil, insbesondere lagerstabil, ist.

8. Zusammensetzung nach einem der vorangehenden Ansprüche,
wobei Zusammensetzung einen Gehalt an Abbauprodukt(en) des Wirkstoffs (a) bzw. der Komponente (a), insbesondere Verunreinigung A, von höchstens 10 Gew.-%, insbesondere von höchstens 5 Gew.-%, vorzugsweise von höchstens 3 Gew.-%, bevorzugt von höchstens 2 Gew.-%, besonders bevorzugt von höchstens 1 Gew.-%, ganz besonders bevorzugt von höchstens 0,9 Gew.-%, bezogen auf den Wirkstoff (a), enthält, insbesondere auch nach Lagerung der Zusammensetzung bei Temperaturen im Bereich von 20 °C bis 50 °C, bei einem Druck von 1.013,25 mbar und bei einer relativen Luftfeuchtigkeit im Bereich von 50 % bis 90 % von mindestens 6 Monaten, insbesondere mindestens 12 Monaten, vorzugsweise mindestens 24 Monaten, bevorzugt mindestens 36 Monaten, besonders bevorzugt mindestens 39 Monaten, ganz besonders bevorzugt mindestens 42 Monaten; und/oder
wobei die Zusammensetzung einen Gehalt an Abbauprodukt(en) des Wirkstoffs (b) bzw. der Komponente (b), insbesondere Aminopropanol und/oder D-Pantolacton, von jeweils höchstens 10 Gew.-%, insbesondere von jeweils höchstens 5 Gew.-%, vorzugsweise von jeweils höchstens 3 Gew.-%, bevorzugt von jeweils höchstens 2 Gew.-%, besonders bevorzugt von jeweils höchstens 1 Gew.-%, ganz besonders bevorzugt von höchstens 0,9 Gew.-%, bezogen auf den Wirkstoff (b), enthält, insbesondere auch nach Lagerung der Zusammensetzung bei Temperaturen im Bereich von 20 °C bis 50 °C, bei einem Druck von 1.013,25 mbar und bei einer relativen Luftfeuchtigkeit im Bereich von 50 % bis 90 % von mindestens 6 Monaten, insbesondere mindestens 12 Monaten, vorzugsweise mindestens 24 Monaten, bevorzugt mindestens 36 Monaten, besonders bevorzugt mindestens 39 Monaten, ganz besonders bevorzugt mindestens 42 Monaten.

9. Zusammensetzung nach einem der vorangehenden Ansprüche zur Verwendung bei der prophylaktischen und/oder kurativen topischen Behandlung von Rhinitis.

10. Applikationsvorrichtung, insbesondere für die topische, insbesondere nasale, vorzugsweise intranasale Applikation, bevorzugt in Form eines Behältnisses mit Tropf- oder Sprüheinrichtung, enthaltend eine Zusammensetzung nach einem der vorangehenden Ansprüche.

11. Verpackungseinheit, enthaltend mindestens eine Applikationsvorrichtung nach Anspruch 10, insbesondere wobei die Aufbewahrungs- und/oder Applikationsvorrichtung in einer vor Kontaminationen schützenden Umverpackung eingebracht vorliegt.

12. Hyaluronsäure und/oder physiologisch verträgliches Hyaluronsäure-Salz (Hyaluronat) zur Verwendung bei der topischen, insbesondere nasalen, bevorzugt intranasalen Behandlung von Rhinitis,
wobei die Hyaluronsäure und/oder das physiologisch verträgliche Hyaluronsäure-Salz (Hyaluronat) in Kombination und/oder in gemeinsamer Zusammensetzung mit
(a) Xylometazolin, insbesondere in Form von dessen physiologisch verträglichen Salzen, besonders bevorzugt Xylometazolinhydrochlorid (Komponente (a)); und
(b) Pantothenol oder dessen physiologisch unbedenkliche Ester (Komponente (b));
vorliegt und/oder eingesetzt wird,
wobei die Hyaluronsäure und/oder das physiologisch verträgliche Hyaluronsäure-Salz ein zahlenmittleres Molekulargewicht (Molmasse) Mₙ im Bereich von 0,5 MDa bis 5 MDa und/oder ein gewichtsmittleres Molekulargewicht (Molmasse) M_{w} im Bereich von 0,6 MDa bis 6 MDa aufweist.

13. Verfahren zur Stabilisierung und/oder zur Verbesserung der Verträglichkeit und/oder zur Erhöhung der Wirksamkeit einer Zusammensetzung, insbesondere pharmazeutischen Zusammensetzung, insbesondere wobei die Zusammensetzung als wässrige Zusammensetzung vorliegt,
wobei die Zusammensetzung in Kombination und jeweils in wirksamen, insbesondere pharmazeutisch wirksamen Mengen
(a) Xylometazolin, insbesondere in Form von dessen physiologisch verträglichen Salzen, besonders bevorzugt Xylometazolinhydrochlorid (Komponente (a));
(b) Pantothenol oder dessen physiologisch unbedenkliche Ester (Komponente (b));
enthält,
durch Zugabe von (c) Hyaluronsäure und/oder eines physiologisch verträglichen Hyaluronsäure-Salzes (Hyaluronat) (Komponente (c)) zu der Zusammensetzung,
wobei die Hyaluronsäure und/oder das physiologisch verträgliche Hyaluronsäure-Salz (Komponente (c)) ein zahlenmittleres Molekulargewicht (Molmasse) Mₙ im Bereich von 0,5 MDa bis 5 MDa und/oder ein gewichtsmittleres Molekulargewicht (Molmasse) M_{w} im Bereich von 0,6 MDa bis 6 MDa aufweist.

14. Verfahren zur Stabilisierung und/oder zur Verbesserung der Verträglichkeit und/oder zur Erhöhung der Wirksamkeit einer Komponente (a) (Wirkstoff (a)) und/oder einer Komponente (b) (Wirkstoff (b)) in einer Zusammensetzung, insbesondere pharmazeutischen Zusammensetzung, insbesondere wobei die Zusammensetzung als wässrige Zusammensetzung vorliegt,
wobei die Komponente (a) Xylometazolin, insbesondere in Form von dessen physiologisch verträglichen Salzen, besonders bevorzugt Xylometazolinhydrochlorid, ist und
wobei die Komponente (b) Pantothenol oder dessen physiologisch unbedenkliche Ester ist,
durch Zugabe von (c) Hyaluronsäure und/oder eines physiologisch verträglichen Hyaluronsäure-Salzes (Hyaluronat) (Komponente (c)), zu der Zusammensetzung,
wobei die Hyaluronsäure und/oder das physiologisch verträgliche Hyaluronsäure-Salz (Komponente (c)) ein zahlenmittleres Molekulargewicht (Molmasse) Mₙ im Bereich von 0,5 MDa bis 5 MDa und/oder ein gewichtsmittleres Molekulargewicht (Molmasse) M_{w} im Bereich von 0,6 MDa bis 6 MDa aufweist.

15. Verwendung von Hyaluronsäure und/oder eines physiologisch verträglichen Hyaluronsäure-Salzes (Hyaluronat)
zur Stabilisierung und/oder zur Verbesserung der Verträglichkeit und/oder zur Erhöhung der Wirksamkeit einer Zusammensetzung, insbesondere pharmazeutischen Zusammensetzung, insbesondere wobei die Zusammensetzung als wässrige Zusammensetzung vorliegt,
wobei die Zusammensetzung in Kombination und jeweils in wirksamen, insbesondere pharmazeutisch wirksamen Mengen
(a) Xylometazolin, insbesondere in Form von dessen physiologisch verträglichen Salzen, besonders bevorzugt Xylometazolinhydrochlorid (Komponente (a));
(b) Pantothenol oder dessen physiologisch unbedenkliche Ester (Komponente (b));
enthält,
wobei die Hyaluronsäure und/oder das physiologisch verträgliche Hyaluronsäure-Salz (Hyaluronat) zu der Zusammensetzung zugegeben wird,
wobei die Hyaluronsäure und/oder das physiologisch verträgliche Hyaluronsäure-Salz (Komponente (c)) ein zahlenmittleres Molekulargewicht (Molmasse) Mₙ im Bereich von 0,5 MDa bis 5 MDa und/oder ein gewichtsmittleres Molekulargewicht (Molmasse) M_{w} im Bereich von 0,6 MDa bis 6 MDa aufweist.

## Claims

1. A composition, in particular a pharmaceutical composition, preferably for topical, in particular nasal, preferably intranasal, application, in particular for the treatment of rhinitis, wherein the composition contains, in combination and in effective, in particular pharmaceutically effective, quantities in each case:
(a) xylometazoline, in particular in the form of its physiologically compatible salts, particularly preferably xylometazoline hydrochloride (component (a));
(b) pantothenol or its physiologically harmless esters (component (b));
(c) hyaluronic acid and/or a physiologically compatible hyaluronic acid salt (hyaluronate) (component (c)),
wherein the hyaluronic acid and/or the physiologically compatible hyaluronic acid salt (component (c)) has a number-average molecular weight (molar mass) Mₙ in the range of 0.5 MDa to 5 MDa and/or a weight-average molecular weight (molar mass) M_{w} in the range of 0.6 MDa to 6 MDa.

2. The composition according to claim 1,
wherein the composition contains component (c) and/or the hyaluronic acid and/or the physiologically compatible hyaluronic acid salt in a quantity in the range of 0.0001 to 5% by weight, in particular in the range of 0.0005 to 3% by weight, preferably in the range of 0.001 to 1% by weight, more preferably in the range of 0.005 to 0.5% by weight, particularly preferably in the range of 0.01 to 0.1% by weight, based on the composition; and/or wherein component (c) and/or the hyaluronic acid and/or the physiologically compatible hyaluronic acid salt has a number-average molecular weight (molar mass) Mₙ in the range of 0.6 MDa to 4.5 MDa, in particular in the range of 0.7 MDa to 4 MDa, preferably in the range of 0.75 MDa to 3.5 MDa, more preferably in the range of 0.8 MDa to 3.25 MDa, particularly preferably in the range of 0.9 MDa to 3 MDa; and/or
wherein component (c) and/or the hyaluronic acid and/or the physiologically compatible hyaluronic acid salt has a weight-average molecular weight (molar mass) M_{w} in the range of 0.7 MDa to 5.5 MDa, in particular in the range of 0.8 MDa to 5 MDa, preferably in the range of 0.85 MDa to 4.5 MDa, more preferably in the range of 0.9 MDa to 4 MDa, particularly preferably in the range of 0.95 MDa to 3 MDa; and/or
wherein component (c) and/or the hyaluronic acid and/or the physiologically compatible hyaluronic acid salt has a polydispersity index (PDI), calculated as the quotient of the weight-average molecular weight M_{w} and the number-average molecular weight Mₙ, in the range of 0.9 to 10, in particular in the range of 0.95 to 5, preferably in the range of 1 to 3, more preferably in the range of 1.05 to 2, particularly preferably in the range of 1.1 to 1.5; and/or
wherein the hyaluronic acid and/or the physiologically compatible hyaluronic acid salt (component (c)) is in the form of sodium hyaluronate.

3. The composition according to claim 1 or 2,
wherein the composition contains component (a) and/or the xylometazoline in a quantity ranging from 0.001 to 2% by weight, in particular from 0.005 to 1.5% by weight, preferably from 0.01 to 1.2% by weight, more preferably from 0.02 to 1.0% by weight, particularly preferably from 0.03 to 0.5% by weight, still more preferably from 0.04 to 0.2% by weight, even more preferably from 0.045 to 0.175% by weight, based on the composition; and/or wherein the composition contains component (b) and/or pantothenol or its physiologically harmless ester in a quantity ranging from 0.01 to 10% by weight, in particular from 0.1 to 9% by weight, preferably from 0.5 to 8% by weight, more preferably from 1 to 7% by weight, particularly preferably from 2 to 6% by weight, still more preferably from 3 to 6% by weight, even more preferably from 3.5 to 5.5% by weight, based on the composition.

4. The composition according to any one of the preceding claims,
wherein the composition is an aqueous composition and/or wherein the composition is aqueous-based and/or has an aqueous formulation, in particular it is in the form of an aqueous solution or aqueous solubilisation; and/or
wherein the composition is an aqueous system, in particular an aqueous single-phase system, preferably an aqueous solution or aqueous solubilisation, and/or that the composition has an excipient or carrier based on water and/or in the form of water and/or that the composition is a clear, colourless aqueous solution; and/or
wherein the composition is free from preservatives and/or free from disinfectants; and/or wherein the pH value of the composition is set to be in the range of 4.9 to 6.1, in particular from 5.0 to 6.0, preferably from 5.1 to 6.0, more preferably from 5.2 to 5.9, particularly preferably from 5.25 to 5.85.

5. The composition according to any one of the preceding claims,
wherein the pH value of the composition is set by means of at least one chemical buffer system, in particular buffer salt(s) (component (d)); and/or
wherein the composition has at least one chemical buffer system (component (d)).

6. The composition according to claim 5,
wherein the composition contains the chemical buffer system (component (d)) in a quantity in the range of 0.01 to 5% by weight, preferably in the range of 0.05 to 4% by weight, more preferably in the range of 0.1 to 3% by weight, particularly preferably in the range of 0.5 to 2% by weight, based on the composition; and/or
*wherein preferably a dihydrogen phosphate*/*monohydrogen phosphate buffer system* ("*H₂PO₄⁻*/*HPO₄*^{*2*-} *buffer (system)" or "phosphate buffer (system)'), in particular an alkali dihydrogen phosphate*/*alkali monohydrogen phosphate buffer system, is used* as *the chemical buffer system (component (d)); and*/*or*
*wherein the composition preferably contains a dihydrogen phosphate*/*monohydrogen phosphate buffer system ("H₂PO₄⁻*/*HPO₄²⁻ buffer (system)" or "phosphate buffer (system)"), in particular an alkali dihydrogen phosphate*/*alkali monohydrogen phosphate buffer system* as *the chemical buffer system (component (d)); and*/*or*
wherein the dihydrogen phosphate/monohydrogen phosphate buffer system is used with a dihydrogen phosphate/monohydrogen phosphate molar ratio of greater than 5:1, in particular in the range of 5:1 to 110:1, particularly preferably in the range of 6:1 to 105:1, particularly preferably in the range of 7:1 to 100:1, still more preferably in the range from 8:1 to 90:1; and/or
wherein the composition contains the dihydrogen phosphate/monohydrogen phosphate buffer system with a dihydrogen phosphate/monohydrogen phosphate molar ratio of greater than 5:1, in particular in the range of 5:1 to 110:1, particularly preferably in the range of 6:1 to 105:1, particularly preferably in the range of 7:1 to 100:1, still more preferably in the range of 8:1 to 90:1.

7. The composition according to any one of the preceding claims,
wherein the composition is stable, in particular is stable in storage, at temperatures in the range of 20°C to 50°C, at a pressure of 1,013.25 mbar and at a relative air humidity in the range of 50% to 90%, for at least 6 months, in particular at least 12 months, preferably at least 24 months, more preferably at least 36 months, particularly preferably at least 39 months, still more preferably at least 42 months.

8. The composition according to any one of the preceding claims,
wherein the composition has a content of degradation product(s) of the active ingredient (a) or component (a), in particular impurity A, of at most 10% by weight, in particular of at most 5% by weight, preferably of at most 3% by weight, more preferably of at most 2% by weight, particularly preferably of at most 1% by weight, still more preferably of at most 0.9% by weight, based on the active ingredient (a), in particular also following storage of the composition at temperatures in the range of 20°C to 50°C, at a pressure of 1,013.25 mbar and at a relative air humidity in the range of 50% to 90%, for at least 6 months, in particular at least 12 months, preferably at least 24 months, more preferably at least 36 months, particularly preferably at least 39 months, still more preferably at least 42 months; and/or wherein the composition contains a content of degradation product(s) of the active ingredient (b) or component (b), in particular aminopropanol and/or D-pantolactone, of at most 10% by weight in each case, in particular of at most 5% by weight in each case, preferably of at most 3% by weight in each case, more preferably of at most 2% by weight in each case, particularly preferably of at most 1% by weight in each case, still more preferably of at most 0.9% by weight, based on the active ingredient (b), in particular also following storage of the composition at temperatures in the range of 20°C to 50°C, at a pressure of 1,013.25 mbar and at a relative air humidity in the range of 50% to 90%, for at least 6 months, in particular at least 12 months, preferably at least 24 months, more preferably at least 36 months, particularly preferably at least 39 months, still more preferably at least 42 months.

9. The composition according to any one of the preceding claims for use in the prophylactic and/or curative topical treatment of rhinitis.

10. An application device, in particular for topical, in particular nasal, preferably intranasal, application, more preferably in the form of a container having a dropper or spraying means, containing a composition according to any one of the preceding claims.

11. A packaging unit, containing at least one application device according to claim 10, in particular wherein the storage and/or application device is present in outer packaging that protects against contamination.

12. Hyaluronic acid and/or physiologically compatible hyaluronic acid salt (hyaluronate) for use in the topical, in particular nasal, preferably intranasal, treatment of rhinitis,
wherein the hyaluronic acid and/or the physiologically compatible hyaluronic acid salt (hyaluronate) is and/or is used in combination and/or in a joint composition with
(a) xylometazoline, in particular in the form of its physiologically compatible salts, particularly preferably xylometazoline hydrochloride (component (a)); and
(b) pantothenol or its physiologically harmless esters (component (b));
wherein the hyaluronic acid and/or the physiologically compatible hyaluronic acid salt has a number-average molecular weight (molar mass) Mₙ in the range of 0.5 MDa to 5 MDa and/or a weight-average molecular weight (molar mass) M_{w} in the range of 0.6 MDa to 6 MDa.

13. A method for stabilising and/or improving the compatibility and/or increasing the effectiveness of a composition, in particular a pharmaceutical composition, in particular wherein the composition is an aqueous composition,
wherein the composition contains, in combination and in effective, in particular pharmaceutically effective, quantities in each case
(a) xylometazoline, in particular in the form of its physiologically compatible salts, particularly preferably xylometazoline hydrochloride (component (a));
(b) pantothenol or its physiologically harmless esters (component (b));
by adding (c) hyaluronic acid and/or a physiologically compatible hyaluronic acid salt (hyaluronate) (component (c)) to the composition,
wherein the hyaluronic acid and/or the physiologically compatible hyaluronic acid salt (component (c)) has a number-average molecular weight (molar mass) Mₙ in the range of 0.5 MDa to 5 MDa and/or a weight-average molecular weight (molar mass) M_{w} in the range of 0.6 MDa to 6 MDa.

14. A method for stabilising and/or improving the compatibility and/or increasing the effectiveness of a component (a) (active ingredient (a)) and/or a component (b) (active ingredient (b)) in a composition, in particular a pharmaceutical composition, in particular wherein the composition is an aqueous composition,
wherein component (a) is xylometazoline, in particular in the form of its physiologically compatible salts, particularly preferably xylometazoline hydrochloride, and
wherein component (b) is pantothenol or its physiologically harmless esters,
by adding (c) hyaluronic acid and/or a physiologically compatible hyaluronic acid salt (hyaluronate) (component (c)) to the composition,
wherein the hyaluronic acid and/or the physiologically compatible hyaluronic acid salt (component (c)) has a number-average molecular weight (molar mass) Mₙ in the range of 0.5 MDa to 5 MDa and/or a weight-average molecular weight (molar mass) M_{w} in the range of 0.6 MDa to 6 MDa.

15. Use of hyaluronic acid and/or a physiologically compatible hyaluronic acid salt (hyaluronate) for stabilising and/or improving the compatibility and/or increasing the effectiveness of a composition, in particular a pharmaceutical composition, in particular wherein the composition is an aqueous composition,
wherein the composition contains, in combination and in effective, in particular pharmaceutically effective, quantities in each case
(a) xylometazoline, in particular in the form of its physiologically compatible salts, particularly preferably xylometazoline hydrochloride (component (a));
(b) pantothenol or its physiologically harmless esters (component (b));
wherein the hyaluronic acid and/or the physiologically compatible hyaluronic acid salt (hyaluronate) is added to the composition,
wherein the hyaluronic acid and/or the physiologically compatible hyaluronic acid salt (component (c)) has a number-average molecular weight (molar mass) Mₙ in the range of 0.5 MDa to 5 MDa and/or a weight-average molecular weight (molar mass) M_{w} in the range of 0.6 MDa to 6 MDa.

## Revendications

1. Composition, en particulier composition pharmaceutique, de préférence pour application topique, en particulier nasale, de préférence intranasale, en particulier pour le traitement de la rhinite,
la composition contenant en association et dans chaque cas en quantités efficaces, en particulier pharmaceutiquement efficaces :
(a) de la xylométazoline, en particulier sous la forme de ses sels physiologiquement acceptables, plus préférablement le chlorhydrate de xylométazoline (composant (a)) ;
(b) du pantothénol ou ses esters physiologiquement acceptables (composant (b)) ;
(c) de l'acide hyaluronique et/ou un sel d'acide hyaluronique physiologiquement acceptable (hyaluronate) (composant (c)),
l'acide hyaluronique et/ou le sel d'acide hyaluronique physiologiquement acceptable (composant (c)) présentant une masse moléculaire moyenne en nombre (masse molaire) Mₙ de 0,5 MDa à 5 MDa et/ou une masse moléculaire moyenne en poids (masse molaire) M_{w} de 0,6 MDa à 6 MDa.

2. Composition selon la revendication 1,
la composition contenant le composant (c) et/ou l'acide hyaluronique et/ou le sel d'acide hyaluronique physiologiquement acceptable en une quantité située dans la plage de 0,0001 % en poids à 5 % en poids, en particulier dans la plage de 0,0005 % en poids à 3 % en poids, de préférence dans la plage de 0,001 % en poids à 1 % en poids, préférablement dans la plage de 0,005 % en poids à 0,5 % en poids, plus préférablement dans la plage de 0,01 % en poids à 0,1 % en poids par rapport à la composition ; et/ou le composant (c) et/ou l'acide hyaluronique et/ou le sel d'acide hyaluronique physiologiquement acceptable présentant une masse moléculaire moyenne en nombre (masse molaire) Mₙ de 0,6 MDa à 4,5 MDa, en particulier dans la plage de 0,7 MDa à 4 MDa, de préférence dans la plage de 0,75 MDa à 3,5 MDa, préférablement dans la plage de 0,8 MDa à 3,25 MDa, plus préférablement dans la plage de 0,9 MDa à 3 MDa ; et/ou le composant (c) et/ou l'acide hyaluronique et/ou le sel d'acide hyaluronique physiologiquement acceptable présentant une masse moléculaire moyenne en poids (masse molaire) M_{w} située dans la plage de 0,7 MDa à 5,5 MDa, en particulier dans la plage de 0,8 MDa à 5 MDa, de préférence dans la plage de 0,85 MDa à 4,5 MDa, préférablement dans la plage de 0,9 MDa à 4 MDa, plus préférablement dans la plage de 0,95 MDa à 3 MDa ; et/ou
le composant (c) et/ou l'acide hyaluronique et/ou le sel d'acide hyaluronique physiologiquement acceptable présentant un indice de polydispersité (PDI), calculé en tant que rapport de la masse moléculaire moyenne en poids M_{w} et de la masse moléculaire moyenne en nombre Mₙ, dans la plage de 0,9 à 10, en particulier dans la plage de 0,95 à 5, de préférence dans la plage de 1 à 3, préférablement dans la plage de 1,05 à 2, plus préférablement dans la plage de 1,1 à 1,5 ;et/ou
l'acide hyaluronique et/ou le sel d'acide hyaluronique physiologiquement acceptable (composant (c)) étant sous la forme de hyaluronate de sodium.

3. Composition selon la revendication 1 ou 2,
la composition contenant le composant (a) et/ou la xylométazoline en une quantité située dans la plage de 0,001 à 2 % en poids, en particulier dans la plage de 0,005 % en poids à 1,5 % en poids, de préférence dans la plage de 0,01 % en poids à 1,2 % en poids, préférablement dans la plage de 0,02 % en poids à 1,0 % en poids, plus préférablement dans la plage de 0,03 % en poids à 0,5 % en poids, le plus préférablement dans la plage de 0,04 % en poids à 0,2 % en poids, encore plus préférablement dans la plage de 0,045 % en poids à 0,175 % en poids par rapport à la composition ; et/ou la composition contenant le composant (b) et/ou le pantothénol ou ses esters physiologiquement acceptables en une quantité située dans la plage de 0,01 % en poids à 10 % en poids, en particulier dans la plage de 0,1 % en poids à 9 % en poids, de préférence dans la plage de 0,5 % en poids à 8 % en poids, préférablement dans la plage de 1 % en poids à 7 % en poids, plus préférablement dans la plage de 2 % en poids à 6 % en poids, le plus préférablement dans la plage de 3 % en poids à 6 % en poids, encore plus préférablement dans la plage de 3,5 % en poids à 5,5 % en poids par rapport à la composition.

4. Composition selon l'une quelconque des revendications précédentes,
la composition étant présente sous la forme d'une composition aqueuse et/ou la composition ayant une base aqueuse et/ou se présentant sous la forme d'une formulation aqueuse, en particulier sous la forme d'une solution aqueuse ou d'une solubilisation aqueuse ; et/ou
la composition étant présente sous forme de système aqueux, en particulier sous la forme de système aqueux monophasique, de préférence sous la forme de solution aqueuse ou de solubilisation aqueuse, et/ou en ce que la composition présente un excipient ou support à base d'eau et/ou sous la forme d'eau et/ou en ce que la composition est présente sous la forme d'une solution aqueuse limpide incolore ; et/ou
la composition étant exempte de conservateurs et/ou exempte de désinfectants ; et/ou la composition ayant un pH situé dans la plage de 4,9 à 6,1, en particulier dans la plage de 5,0 à 6,0, de préférence dans la plage de 5,1 à 6,0, préférablement dans la plage de 5,2 à 5,9, plus préférablement dans la plage de 5,25 à 5,85.

5. Composition selon l'une quelconque des revendications précédentes,
le pH de la composition étant ajusté au moyen d'au moins un système tampon chimique, en particulier de sel(s) tampon(s) (composant (d)) ; et/ou
la composition présentant au moins un système tampon chimique (composant (d)).

6. Composition selon la revendication 5,
la composition contenant le système tampon chimique (composant (d)) en une quantité située dans la plage de 0,01 % en poids à 5 % en poids, en particulier dans la plage de 0,05 % en poids à 4 % en poids, de préférence dans la plage de 0,1 % en poids à 3 % en poids, plus préférablement dans la plage de 0,5 à 2 % en poids, par rapport à la composition ; et/ou
*un système tampon dihydrogénophosphate*/*monohydrogénophosphate (système tampon H₂PO₄⁻*/*HPO₄*^{*2*-} *ou système tampon phosphate), en particulier un système tampon dihydrogénophosphate alcalin*/*monohydrogénophosphate alcalin étant de préférence utilisé en tant que système tampon chimique (composant (d))* ; *et*/*ou la composition contenant, en tant que système tampon chimique (composant (d)), de préférence un système tampon dihydrogénophosphate*/*monohydrogénophosphate (système tampon H₂PO₄⁻*/*HPO₄*^{*2*-} *ou système tampon phosphate), en particulier un système tampon dihydrogénophosphate alcalin*/*monohydrogénophosphate alcalin ; et*/*ou* le système tampon dihydrogénophosphate/monohydrogénophosphate étant utilisé selon un rapport molaire dihydrogénophosphate/monohydrogénophosphate supérieur à 5: 1, en particulier dans la plage de 5 : 1 à 110 : 1, préférablement dans la plage de 6 : 1 à 105 : 1, plus préférablement dans la plage de 7 : 1 à 100 : 1, encore plus préférablement dans la plage de 8 : 1 à 90 : 1 ; et/ou
la composition contenant le système tampon dihydrogénophosphate/monohydrogénophosphate selon un rapport molaire dihydrogénophosphate/monohydrogénophosphate supérieur à 5 : 1, en particulier dans la plage de 5 : 1 à 110 : 1, préférablement dans la plage de 6 : 1 à 105 : 1, plus préférablement dans la plage de 7 : 1 à 100 : 1, encore plus préférablement dans la plage de 8 : 1 à 90 : 1.

7. Composition selon l'une quelconque des revendications précédentes,
la composition étant stable, en particulier stable au stockage, à des températures dans la plage de 20 °C à 50 °C, à une pression de 1013,25 mbar et à une humidité relative de l'air dans la plage de 50 % à 90 % , pendant au moins 6 mois, en particulier au moins 12 mois, de préférence au moins 24 mois, préférablement au moins 36 mois, plus préférablement au moins 39 mois, encore plus préférablement au moins 42 mois.

8. Composition selon l'une quelconque des revendications précédentes,
la composition ayant une teneur en produit(s) de dégradation du principe actif (a) ou du composant (a), en particulier l'impureté A, d'au plus 10 % en poids, en particulier d'au plus 5 % en poids, de préférence d'au plus 3 % en poids, préférablement d'au plus 2 % en poids, plus préférablement d'au plus 1 % en poids, encore plus préférablement d'au plus 0,9 % en poids, par rapport au principe actif (a), en particulier également après stockage de la composition à des températures dans la plage de 20 °C à 50 °C, à une pression de 1013,25 mbar et à une humidité relative dans la plage de 50 % à 90 % pendant au moins 6 mois, en particulier au moins 12 mois, de préférence au moins 24 mois, préférablement au moins 36 mois, plus préférablement au moins 39 mois, encore plus préférablement 42 mois ; et/ou
la composition ayant une teneur en produit(s) de dégradation du principe actif (b) ou du composant (b), en particulier l'aminopropanol et/ou la D-pantolactone, d'au plus 10 % en poids chacun, en particulier d'au plus 5 % en poids chacun , de préférence d'au plus 3 % en poids chacun, préférablement d'au plus 2 % en poids chacun, plus préférablement d'au plus 1 % en poids chacun, encore plus préférablement d'au plus 0,9 % en poids, par rapport au principe actif (b), en particulier après stockage de la composition à des températures dans la plage de 20 °C à 50 °C, à une pression de 1013,25 mbar et à une humidité relative dans la plage de 50 % à 90 % pendant au moins 6 mois, en particulier au moins 12 mois, de préférence au moins 24 mois, préférablement au moins 36 mois, plus préférablement au moins 39 mois, encore plus préférablement au moins 42 mois.

9. Composition selon l'une quelconque des revendications précédentes, à utiliser dans le traitement topique prophylactique et/ou curatif de la rhinite.

10. Dispositif d'application, en particulier pour l'application topique, en particulier nasale, de préférence intranasale, préférablement sous la forme d'un contenant doté d'un compte-goutte ou d'un pulvérisateur, contenant une composition selon l'une quelconque des revendications précédentes.

11. Unité de conditionnement contenant au moins un dispositif d'application selon la revendication 10, en particulier le dispositif de stockage et/ou d'application étant inséré dans un emballage extérieur protégeant contre toute contamination.

12. Acide hyaluronique et/ou sel d'acide hyaluronique physiologiquement acceptable (hyaluronate) à utiliser dans le traitement topique, en particulier nasal, de préférence intranasal, de la rhinite,
l'acide hyaluronique et/ou le sel d'acide hyaluronique physiologiquement acceptable (hyaluronate) étant présent et/ou utilisé en association et/ou en composition conjointe avec
(a) de la xylométazoline, en particulier sous la forme de ses sels physiologiquement acceptables, plus préférablement le chlorhydrate de xylométazoline (composant (a)) ; et
(b) du pantothénol ou ses esters physiologiquement acceptables (composant (b)) ;
l'acide hyaluronique et/ou le sel d'acide hyaluronique physiologiquement acceptable présentant une masse moléculaire moyenne en nombre (masse molaire) Mₙ de 0,5 MDa à 5 MDa et/ou une masse moléculaire moyenne en poids (masse molaire) M_{w} de 0,6 MDa à 6 à MDa.

13. Procédé pour stabiliser et/ou améliorer la compatibilité et/ou augmenter l'efficacité d'une composition, en particulier d'une composition pharmaceutique, en particulier lorsque la composition se présente sous la forme d'une composition aqueuse,
la composition en association et respectivement en des quantités efficaces, en particulier pharmaceutiquement efficaces, contenant
(a) de la xylométazoline, en particulier sous la forme de ses sels physiologiquement acceptables, plus préférablement le chlorhydrate de xylométazoline (composant (a)) ;
(b) du pantothénol ou ses esters physiologiquement acceptables (composant (b)) ;
grâce à l'ajout (c) d'acide hyaluronique et/ou d'un sel d'acide hyaluronique physiologiquement acceptable (hyaluronate) (composant (c)) à la composition,
l'acide hyaluronique et/ou le sel d'acide hyaluronique physiologiquement acceptable (composant (c)) présentant une masse moléculaire moyenne en nombre (masse molaire) Mₙ de 0,5 MDa à 5 MDa et/ou une masse moléculaire moyenne en poids (masse molaire) M_{w} de 0,6 MDa à 6 MDa.

14. Procédé pour stabiliser et/ou améliorer la compatibilité et/ou augmenter l'efficacité d'un composant (a) (principe actif (a)) et/ou d'un composant (b) (principe actif (b)) dans une composition, en particulier une composition pharmaceutique, en particulier lorsque la composition se présente sous la forme d'une composition aqueuse,
le composant (a) étant la xylométazoline, en particulier sous la forme de ses sels physiologiquement acceptables, plus préférablement le chlorhydrate de xylométazoline, et le composant (b) étant le pantothénol ou ses esters physiologiquement acceptables, grâce à l'ajout (c) d'acide hyaluronique et/ou d'un sel d'acide hyaluronique physiologiquement acceptable (hyaluronate) (composant (c)) à la composition,
l'acide hyaluronique et/ou le sel d'acide hyaluronique physiologiquement acceptable (composant (c)) présentant une masse moléculaire moyenne en nombre (masse molaire) Mₙ de 0,5 MDa à 5 MDa et/ou une masse moléculaire moyenne en poids (masse molaire) M_{w} de 0,6 MDa à 6 MDa.

15. Utilisation d'acide hyaluronique et/ou d'un sel d'acide hyaluronique physiologiquement acceptable (hyaluronate)
pour stabiliser et/ou améliorer la compatibilité et/ou augmenter l'efficacité d'une composition, en particulier d'une composition pharmaceutique, en particulier lorsque la composition se présente sous la forme d'une composition aqueuse,
la composition en association et respectivement en des quantités efficaces, en particulier pharmaceutiquement efficaces, contenant
(a) de la xylométazoline, en particulier sous la forme de ses sels physiologiquement acceptables, plus préférablement le chlorhydrate de xylométazoline (composant (a)) ;
(b) du pantothénol ou ses esters physiologiquement acceptables (composant (b)) ;
l'acide hyaluronique et/ou le sel d'acide hyaluronique physiologiquement acceptable (hyaluronate) étant ajouté à la composition,
l'acide hyaluronique et/ou le sel d'acide hyaluronique physiologiquement acceptable (composant (c)) présentant une masse moléculaire moyenne en nombre (masse molaire) Mₙ de 0,5 MDa à 5 MDa et/ou une masse moléculaire moyenne en poids (masse molaire) M_{w} de 0,6 MDa à 6 MDa.
